# EUROPEAN PATENT SPECIFICATION

(11) **EP 4 290 479 B1**
(45) Date of publication and mention of the grant of the patent: **04.11.2026**
(21) Application number: 22305840.5
(22) Date of filing: 09.06.2022
(51) Int. Cl.: G06V 30/14, G06V 30/146, G06V 30/148, G06V 30/262

(54) **METHOD AND SYSTEM FOR READING AN OPTICAL PRESCRIPTION ON AN OPTICAL PRESCRIPTION IMAGE**
VERFAHREN UND SYSTEM ZUM LESEN EINER OPTISCHEN VERSCHREIBUNG AUF EINEM OPTISCHEN REZEPTBILD
PROCÉDÉ ET SYSTÈME PERMETTANT DE LIRE UNE PRESCRIPTION OPTIQUE SUR UNE IMAGE DE PRESCRIPTION OPTIQUE

(43) Date of publication of application: 13.12.2023
(73) Proprietor: Essilor International, 94220 Charenton-Le-Pont (FR)
(72) Inventor: TOH, Yin Zhen Janice, 649812 Singapore (SG); GARCIN, Gilles, 426927 Singapore (SG); NG, Jinhhao, 570443 Singapore (SG)
(74) Representative: Jacobacci & Partners France

(56) References cited:
- KUMAR RAI BIPIN BIPINKRAI@GMAIL COM ET AL: "Medical Prescription and Report Analyzer", ADJUNCT PROCEEDINGS OF THE 30TH ACM CONFERENCE ON USER MODELING, ADAPTATION AND PERSONALIZATION, ACMPUB27, NEW YORK, NY, USA, 5 August 2021 (2021-08-05), pages 286 - 295, XP058812287, ISBN: 978-1-4503-9668-4, DOI: 10.1145/3474124.3474165
- HASSAN ESRAA ET AL: "Medical Prescription Recognition using Machine Learning", 2021 IEEE 11TH ANNUAL COMPUTING AND COMMUNICATION WORKSHOP AND CONFERENCE (CCWC), IEEE, 27 January 2021 (2021-01-27), pages 973 - 979, XP033887674, [retrieved on 20210310], DOI: 10.1109/CCWC51732.2021.9376141

## Description

### TECHNICAL FIELD

Various aspects of this disclosure relate to a method for reading an optical prescription on an optical prescription image. Various aspects of this disclosure further relate to a system for reading an optical prescription on an optical prescription image.

### BACKGROUND

The practice of recording an optical prescription, in particular, a handwritten optical prescription or handwritten optical prescription on a prescription form is not standardized and is largely dependent on the individual practices of the eye care practitioner who is recording the optical prescription. The optical prescription thus varies in format and is often unclear to a user, in particular, the subject or parents who may wish to understand and/or monitor their or their child(ren)'s visual health and myopia evolution.

Conventional optical character recognition (OCR) technologies may be used to convert the optical prescription, e.g. a handwritten optical prescription, into text. However, the conversion of such optical prescriptions is often inaccurate, especially since the accuracy of the converted optical prescription is largely dependent on the OCR technology employed. Further, the text of the converted optical prescription is usually in the form of an incoherent string of numbers, alphabetic characters and/or symbols. The eye care practitioner thus has to manually classify the incoherent string of text into specific optical categories, and to manually correct the errors in the text of the converted optical prescription. The process of manually classifying and correcting the errors in the text of the of the converted optical prescription is inefficient and cumbersome, and requires a trained professional such as an eye care practitioner.

KUMAR RAI BIPIN BIPINKRAI@GMAIL COM ET AL: "Medical Prescription and Report Analyzer", ADJUNCT PROCEEDINGS OF THE 30TH ACM CONFERENCE ON USER MODELING, ADAPTATION AND PERSONALIZATION, ACMPUB27, NEW YORK, NY, USA, 5 August 2021 (2021-08-05), pages 286-295, DOI: 10.1145/3474124.3474165 ISBN: 978-1-4503-9668-4, discloses a method for medical prescription analysis.

HASSAN ESRAA ET AL: "Medical Prescription Recognition using Machine Learning",2021 IEEE 11TH ANNUAL COMPUTING AND COMMUNICATION WORKSHOP AND CONFERENCE (CCWC), IEEE, 27 January 2021 (2021-01-27), pages 973-979, DOI: 10.1109/ CCWC51732.2021.9376141, discloses a method for medical prescription recognition.

Thus, it is desired to seek more accurate and efficient means to read an optical prescription on an optical prescription image.

### SUMMARY

The invention is defined by the set of appended claims. It is an object of the disclosure to provide methods and a system to accurately and efficiently read an optical prescription on an optical prescription image. To this end, methods and a system are provided for the extraction of an optical prescription and conversion thereof into optical prescription data; classification of the optical prescription data into one or more predetermined categories, to generate an optical prescription value associated with the one or more predetermined categories; and the determination of whether the optical prescription value associated with the one of the one or more predetermined categories contains an error, and, if the optical prescription value contains the error, the correction of the error within the optical prescription value, to generate a corrected optical prescription value. The methods may be implemented on a computing system, to provide the automated extraction, conversion, classification and/or correction of the optical prescription. The methods and system of the disclosure may aid the user in the understanding and/or monitoring of their visual health, in particular, for the myopia onset or progression.

The methods and system are particularly applicable for reading optical prescriptions for myopia management, specifically myopia onset or progression, but can be used for reading all types of optical prescriptions on an optical prescription image.

A first aspect of the disclosure concerns a method for reading an optical prescription on an optical prescription image including: (i.) detecting a region comprising the optical prescription on the optical prescription image; (ii.) extracting the optical prescription and converting the optical prescription into machine-encoded optical prescription data; (iii.) classifying a portion of the optical prescription data into one or more predetermined categories, to generate an optical prescription value associated with a respective one of the one or more predetermined categories; and (iv.) determining whether the optical prescription value associated with the respective one of the one or more predetermined categories contains an error, and, if the optical prescription value contains the error, correcting the error within the optical prescription value, to generate a corrected optical prescription value associated with the respective one of the one or more predetermined categories.

In various embodiments of the first aspect, (i.) detecting the region comprising the optical prescription on the optical prescription image comprises detecting at least one keyword and the portion of the optical prescription data associated with the at least one keyword; and (ii.) extracting the optical prescription comprises extracting the at least one keyword and the portion of the optical prescription data associated with the at least one keyword.

In various embodiments of the first aspect, (i.) detecting the at least one keyword comprises detecting the at least one keyword and a corresponding one or more erroneous keywords associated with a respective one of the at least one keyword.

In various embodiments of the first aspect, wherein the one or more predetermined categories comprises (i.) a first category associated with a subject's spherical data; (ii.) a second category associated with the subject's cylinder data; (iii.) a third category associated with the subject's cylinder axis data; (iv.) a fourth category associated with the subject's additional lens power data; (v.) a fifth category associated with the subject's pupil distance data; and (vi.) a sixth category associated with the subject's axial length data.

In various embodiments of the first aspect, the method further comprises (i.) modifying the optical prescription data such that each portion of the optical prescription data which is to be classified into either of the one or more predetermined categories is separated by a single separator.

In various embodiments of the first aspect, classifying the portion of the optical prescription data into the first category associated with the subject's spherical data comprises (i.) detecting a first punctuation mark in the optical prescription data; and (ii.) extracting a first character string positioned before the first punctuation mark, and a second character string positioned after the first punctuation mark, wherein the first and second character strings each have a first predefined length.

In various embodiments of the first aspect, classifying the portion of the optical prescription data into the second category associated with the subject's cylinder data comprises (i.) detecting a second punctuation mark in the optical prescription data, the second punctuation mark positioned after the first punctuation mark; and (ii.) extracting a third character string positioned before the second punctuation mark, and a fourth character string positioned after the second punctuation mark, wherein the third and fourth character strings each have the first predefined length.

In various embodiments of the first aspect, classifying the portion of the optical prescription into the fourth category associated with the subject's additional lens power data comprises (i.) detecting a third punctuation mark in the optical prescription data, the third punctuation mark positioned after each of the first and second punctuation marks; and (ii.) extracting a fifth character string positioned before the third punctuation mark, and a sixth character string positioned after the third punctuation mark, wherein the fifth and sixth character strings each have the first predefined length.

In various embodiments of the first aspect, the first predefined length comprises 3 characters.

In various embodiments of the first aspect, classifying the portion of the optical prescription data into the sixth category associated with the subject's axial length data comprises (i.) detecting a fourth punctuation mark in the optical prescription data, the fourth punctuation mark positioned after each of the first, second and third punctuation marks; and (ii.) extracting a seventh character string positioned before the fourth punctuation mark, and an eighth character string positioned after the fourth punctuation mark, wherein the seventh and eighth character strings each comprise 2 characters.

In various embodiments of the first aspect, classifying the portion of the optical prescription data into the first category associated with the subject's spherical data comprises (i.) detecting a first keyword from among the at least one keyword, the first keyword associated with the first category; and (ii.) extracting a ninth character string positioned after the first keyword, wherein the ninth character string has a second predefined length.

In various embodiments of the first aspect, classifying the portion of the optical prescription data into the second category associated with the subject's cylinder data comprises (i.) detecting a tenth character string positioned after the portion of the optical prescription data classified into the first category; and (ii.) extracting the tenth character string, wherein the tenth character string has the second predefined length.

In various embodiments of the first aspect, classifying the portion of the optical prescription into the fourth category associated with the subject's additional lens power data comprises (i.) detecting a second keyword from among the at least one keyword, the second keyword associated with the fourth category; and (ii.) extracting an eleventh character string positioned after the second keyword, wherein the eleventh character string has the second predefined length.

In various embodiments of the first aspect, classifying the portion of the optical prescription into the sixth category associated with the subject's axial length data comprises (i.) detecting a third keyword from among the at least one keyword, the third keyword associated with the sixth category; and (ii.) extracting a twelfth character string positioned after the third keyword, wherein the twelfth character string has the second predefined length.

In various embodiments of the first aspect, the second predefined length comprises 7 characters.

In various embodiments of the first aspect, classifying the portion of the optical prescription data into the first category associated with the subject's spherical data comprises (i.) detecting a first symbol and a first separator in the optical prescription data; and (ii.) extracting a thirteenth character string positioned between the first symbol and the first separator.

In various embodiments of the first aspect, classifying the portion of the optical prescription data into the second category associated with the subject's cylinder data comprises (i.) detecting a second symbol and a second separator in the optical prescription data, the second symbol and second separator positioned after the first symbol and the first separator; and (ii.) extracting a fourteenth character string positioned between the second symbol and the second separator.

In various embodiments of the first aspect, classifying the portion of the optical prescription data into the third category associated with the subject's cylinder axis data comprises (i.) detecting a third separator in the optical prescription data, the third separator positioned after each of the first and second separators; and (ii.) extracting a fifteenth character string positioned after the third separator.

In various embodiments of the first aspect, classifying the portion of the optical prescription into the fourth category associated with the subject's additional lens power data comprises (i.) detecting a third symbol and a fourth separator in the optical prescription data, the third symbol and the fourth separator positioned after each of the first and second symbols and each of the first to third separators; (ii.) extracting a sixteenth character string positioned between the third symbol and the fourth separator.

In various embodiments of the first aspect, classifying the portion of the optical prescription data into the third category associated with the subject's cylinder axis data comprises (i.) detecting a seventeenth character string positioned after the portion of the optical prescription data classified into the second category; and (ii.) extracting the seventeenth character string, wherein the seventeenth character string comprises 6 characters.

In various embodiments of the first aspect, classifying the portion of the optical prescription into the fifth category associated with the subject's pupil distance data comprises (i.) detecting a fourth keyword from among the at least one keyword, the fourth keyword associated with the fifth category; and extracting an eighteenth character string positioned after the fourth keyword, wherein the eighteenth character string comprises 5 characters.

In various embodiments of the first aspect, the method further comprises (i.) determining if a unit associated with the fifth category is present in the eighteenth character string; (ii.) if it is determined that the unit associated with the fifth category is present in the eighteenth character string: extracting a nineteenth character string positioned before the unit associated with the fifth category, to generate the optical prescription value associated with the fifth category.

In various embodiments of the first aspect, the method further comprises (i.) determining if a unit associated with the sixth category is present in the twelfth character string; (ii.) if it is determined that the unit associated with the sixth category is present in the twelfth character string: extracting a twentieth character string positioned before the unit associated with the sixth category, to generate the optical prescription value associated with the sixth category.

In various embodiments of the first aspect, the method further comprises (i.) detecting at least one of a numeral, an alphabetic character, a fourth symbol, a fifth punctuation mark, in each of the first to eighteenth character strings; and (ii.) extracting the at least one of the numeral, the alphabetic character, the fourth symbol, the fifth punctuation mark detected in each of the first to eighteenth character strings, to generate the optical prescription value associated with the respective one of the one or more predetermined categories.

In various embodiments of the first aspect, (i.) the optical prescription value associated with each of the first, second, fourth, and sixth categories has a length less than or equal to 5 characters; (ii.) the optical prescription value associated with the third category has a length less than or equal to 4 characters; and (iii.) the optical prescription value associated with the fifth category has a length less than or equal to 2 characters.

In various embodiments of the first aspect, determining whether the optical prescription value associated with the respective one of the one or more predetermined categories contains the error, and, if the optical prescription value contains the error, correcting the error within the optical prescription value associated with the respective one of the one or more predetermined categories comprises (i.) determining if the at least one alphabetic character is present in the optical prescription value associated with the respective one of the one or more predetermined categories; (ii.) if it is determined that the at least one alphabetic character is present in the optical prescription value associated with the respective one of the one or more predetermined categories: (a.) matching the at least one alphabetic character to one or more predetermined alphabetic character candidates, wherein the one or more predetermined alphabetic character candidates each comprise a corresponding predetermined numeric candidate; (b.) selecting the corresponding predetermined numeric candidate, by basing the selection on the matching of the at least one alphabetic character to the one or more predetermined alphabetic character candidates; and (c.) correcting the at least one alphabetic character present in the optical prescription value associated with the respective one of the one or more predetermined categories, to the selected corresponding predetermined numeric candidate.

In various embodiments of the first aspect, the method further comprises (i.) determining if the fifth punctuation mark is present in the optical prescription value associated with the respective one of the one or more predetermined categories; (ii.) if it is determined that the fifth punctuation mark is absent in the optical prescription value associated with the respective one of the one or more predetermined categories: (a.) detecting a last two numerals in the optical prescription value associated with the respective one of the one or more predetermined categories; and (b.) inserting the fifth punctuation mark prior to the last two numerals of the optical prescription value associated with the respective one of the one or more predetermined categories.

In various embodiments of the first aspect, the method further comprises (i.) determining if the last two numerals of the optical prescription value associated with the respective one of the one or more predetermined categories is identical to one or more predetermined numerical strings; (ii.) if it is determined that the last two numerals of the optical prescription value associated with the respective one of the one or more predetermined categories differs to the one or more predetermined numerical strings: (a.) matching the last two numerals of the optical prescription value associated with the respective one of the one or more predetermined categories to one or more predetermined erroneous numerical strings, wherein the one or more predetermined erroneous numerical strings each comprise a corresponding predetermined numerical string; (b.) selecting the corresponding predetermined numerical string, by basing the selection on the matching of the last two numerals to the one or more predetermined erroneous numerical strings; and (c.) correcting the last two numerals in the optical prescription value associated with the respective one of the one or more predetermined categories, to the selected corresponding predetermined numerical string; (iii.) generating the corrected optical prescription value associated with the respective one of the one or more predetermined categories.

In various embodiments of the first aspect, the method further comprises (i.) determining if the corrected optical prescription value associated with the respective one of the one or more predetermined categories falls within a reference range of values associated with the respective one of the one or more predetermined categories; and (ii.) emitting a first alert, if it is determined that the corrected optical prescription value falls outside the reference range of values associated with the respective one of the one or more predetermined categories.

In various embodiments of the first aspect, the method further comprises (i.) calculating a difference between the corrected optical prescription value associated with the respective one of the one or more predetermined categories with a respective previous optical prescription value associated with the respective one of the one or more predetermined categories; and (ii.) emitting a second alert, if it is determined that the difference is greater than a predefined threshold value associated with the respective one of the one or more predetermined categories.

In various embodiments of the first aspect, correcting the error within the optical prescription value associated with the respective one of the one or more predetermined categories to generate the corrected optical prescription value, is based on a machine learning algorithm.

A second aspect of the disclosure concerns a system for reading an optical prescription on an optical prescription image including: (i.) means for detecting a region comprising the optical prescription on the optical prescription image; (ii.) means for extracting the optical prescription and converting the optical prescription into machine-encoded optical prescription data; (iii.) means for classifying a portion of the optical prescription data into one or more predetermined categories, to generate an optical prescription value associated with a respective one of the one or more predetermined categories; and (iv.) means for determining whether the optical prescription value associated with the respective one of the one or more predetermined categories contains an error, and, if the optical prescription value contains the error, correcting the error within the optical prescription value associated with the respective one of the one or more predetermined categories, to generate a corrected optical prescription value associated with the respective one of the one or more predetermined categories.

In various embodiments of the second aspect, at least one of the means for detecting the region comprising the optical prescription, the means for extracting the optical prescription and converting the optical prescription into machine-encoded optical prescription data, the means for classifying the portion of the optical prescription data into one or more predetermined categories, to generate the optical prescription value associated with a respective one of the one or more predetermined categories, and the means for determining whether the optical prescription value associated with the respective one of the one or more predetermined categories contains the error, and if the optical prescription value contains the error, correcting the error within the optical prescription value associated with the respective one of the one or more predetermined categories, to generate the corrected optical prescription value associated with the respective one of the one or more predetermined categories, comprises a circuit.

In various embodiments of the second aspect, the circuit associated with the means for classifying the portion of the optical prescription data into one or more predetermined categories, to generate the optical prescription value associated with the respective one of the one or more predetermined categories, and the circuit associated with the means for determining whether the optical prescription value associated with the respective one of the one or more predetermined categories contains the error, and if the optical prescription value contains the error, correcting the error within the optical prescription value associated with the respective one of the one or more predetermined categories, to generate the corrected optical prescription value associated with the respective one of the one or more predetermined categories, further comprises a memory configured to store the optical prescription value associated with the respective one of the one or more predetermined categories, and the corrected optical prescription value associated with the respective one of the one or more predetermined categories, and wherein said circuits are further configured to send the optical prescription value associated with the respective one of the one or more predetermined categories, and the corrected optical prescription value associated with the respective one of the one or more predetermined categories, to an external server.

A third aspect of the disclosure concerns a computer program product, comprising instructions to cause the system according to the second aspect to execute the steps of the method according to the first aspect.

According to various embodiments, the optical prescription on the optical prescription image may be a handwritten optical prescription for myopia.

### BRIEF DESCRIPTION OF THE DRAWINGS

The disclosure will be better understood with reference to the detailed description when considered in conjunction with the non-limiting examples and the accompanying drawings, in which:
- Figure 1 shows a schematic illustration of a method 100 for reading an optical prescription on an optical prescription image;
- Figure 2A shows an exemplary erroneous keyword table 200A of the at least one keyword and a corresponding erroneous keyword associated with said at least one keyword; Figure 2B shows an example 200B of the detection, extraction and conversion ("recording") of a handwritten optical prescription on an optical prescription image 210 into optical prescription data 220; and Figure 2C shows another example 200C of the recording of a handwritten optical prescription on a printed prescription form 230 into optical prescription data 240, in accordance with steps 102 and 104 of method 100;
- Figure 3 shows an exemplary optical prescription image 300 where each portion of the optical prescription may be positioned in accordance with the general rules and protocols for writing an optical prescription;
- Figures 4A to 4D show exemplary schematic illustrations of methods 400A, 400B, 400C, 400D for classifying the portion of the optical prescription data into one or more predetermined categories, in accordance with an embodiment of the disclosure;
- Figures 5A to 5F show exemplary schematic illustrations of methods 500A, 500B, 500C, 500D, 500E, 500F for classifying the portion of the optical prescription data into one or more predetermined categories, in accordance with another embodiment of the disclosure;
- Figures 6A to 6D show exemplary schematic illustrations of methods 600A, 600B, 600C, 600D for classifying the portion of the optical prescription data into one or more predetermined categories, in accordance with another embodiment of the disclosure;
- Figures 7A to 7C show exemplary schematic illustrations of methods 700A, 700B, 700C for determining whether the optical prescription value associated with the respective one of the one or more predetermined categories contains an error, and, if the optical prescription value contains the error, correcting the error within the optical prescription value, to generate a corrected optical prescription value associated with the respective one of the one or more predetermined categories, in accordance with the various embodiments of the disclosure;

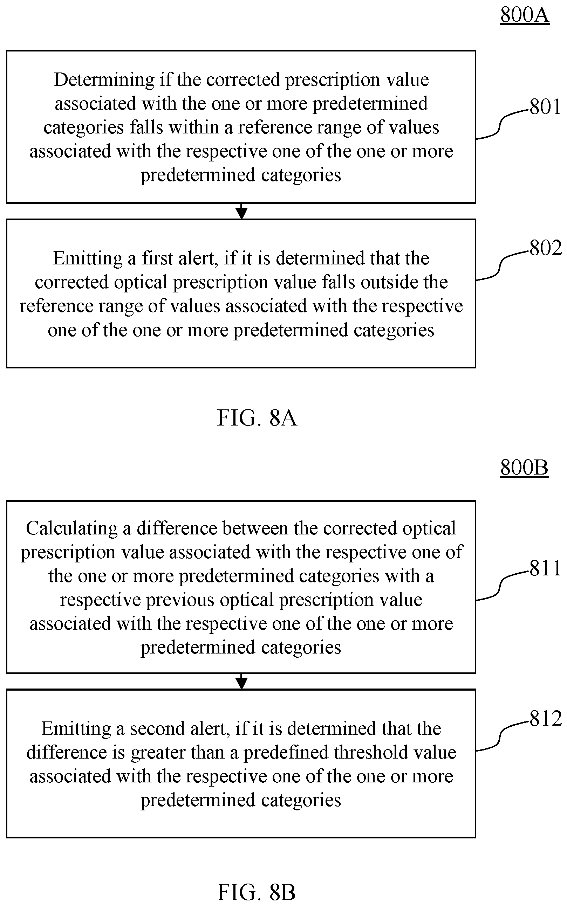

- Figure 8A shows a flowchart of method 800A for determining if the corrected prescription value associated with the one or more predetermined categories falls within a reference range of values associated with the respective one of the one or more predetermined categories; and Figure 8B shows a flowchart of method 800B for determining if a difference between the corrected optical prescription value associated with the respective one of the one or more predetermined categories and a respective previous optical prescription value associated with the respective one of the one or more predetermined categories is greater than a predefined threshold value;
- Figure 9 shows a schematic illustration of a system 900 for reading an optical prescription on an optical prescription image;
- Figure 10 shows an example 1000 for reading a handwritten optical prescription on an optical prescription image 1001, including Chinese characters;
- Figure 11 shows another example 1100 for reading a handwritten optical prescription on an optical prescription image 1101, including additional lens power measurements; and
- Figure 12 shows another example 1200 for reading a handwritten optical prescription on an optical prescription image 1201.

### DETAILED DESCRIPTION

The following detailed description refers to the accompanying drawings that show, by way of illustration, specific details and embodiments in which the disclosure may be practiced. These embodiments are described in sufficient detail to enable those skilled in the art to practice the disclosure. The various embodiments are not necessarily mutually exclusive, as some embodiments can be combined with one or more other embodiments to form new embodiments.

Features that are described in the context of an embodiment may correspondingly be applicable to the same or similar features in the other embodiments. Features that are described in the context of an embodiment may correspondingly be applicable to the other embodiments, even if not explicitly described in these other embodiments. Furthermore, additions and/or combinations and/or alternatives as described for a feature in the context of an embodiment may correspondingly be applicable to the same or similar feature in the other embodiments.

In the context of various embodiments, the articles "a", "an" and "the" as used with regard to a feature or element include a reference to one or more of the features or elements. As used herein, the term "and/or" includes any and all combinations of one or more of the associated listed items.

The reference signs included in parenthesis in the claims are for ease of understanding of the disclosure and have no limiting effect on the scope of the claims.

According to various embodiments, the term "reading", as used herein, refers to a machine-implemented acquisition of information of text containing an optical prescription from an optical prescription image. Within the context of the disclosure, the term "reading" encompasses the detection, extraction, conversion, classification and/or correction of the optical prescription.

According to various embodiments, the term "optical prescription", as used herein, refers broadly to an optical measurement indicative of the visual health of a subject. In some embodiments, the optical prescription may be a measurement for myopia and/or astigmatism of the subject. In some other embodiments, the optical prescription may be a measurement for hyperopia.

According to various embodiments, the term "optical prescription image", as used herein, may refer to a digital image of a prescription form. In some embodiments, the prescription form may be a hard copy prescription form and may include printed and/or handwritten text. The digital image may be acquired by scanning the prescription form using an image sensor. The optical prescription image may have various formats, and a prescription form of an eye care practitioner may differ to that from another eye care practitioner. In some embodiments, the optical prescription on the optical prescription image may be handwritten on a blank piece of paper.

According to various embodiments, the term "subject", as used herein, may refer to any individual undergoing an optical examination for obtaining the optical prescription. In some embodiments, the term "subject" may refer to a child or an adolescent, for example, below the age of 12 years old. In some embodiments, the subject may refer to an emmetropic, e.g. non- or pre-myopic individual, or a myopic or ametropic individual.

According to various embodiments, the term "error", as used herein, may refer to an incorrect conversion of a numeral or an alphabetic character within the extracted optical prescription into machine-encoded optical prescription data. The errors identified by the methods of the present disclosure may have one or more corresponding predetermined erroneous numerals or alphabetic characters associated with the identified error.

According to various embodiments, the term "keyword", as used herein, may refer to a word indicative of the portion of the optical prescription, or the optical prescription data which is associated with the keyword. The keyword may be in any language, for example, the English language, or the Chinese language, e.g. Chinese character.

According to various embodiments, the term "detecting", as used herein, may refer to a machine-enabled optical identification of a keyword or a character, e.g. numeral, alphabetic character, symbol, punctuation mark and/or separator. Detecting the keyword or character may include scanning each row of the optical prescription image from the left-hand side (LHS) to the right-hand side (RHS) of the optical prescription image, starting at the top RHS of the first row of on the optical prescription image.

According to various embodiments, the term "before" and "after" as used herein, may refer to the position of the portion of the optical prescription or character string. The term "before" may refer a portion of the optical prescription or character string which is positioned in front of, preceding or towards the left or LHS; and the term "after" may refer to a portion of the optical prescription or character string which is positioned behind of, succeeding or towards the right or RHS, with reference to another portion, character string and/or character.

According to various embodiments, the term "character string", as used herein, may refer to a series of characters within a string. The character string may refer to a data string as used in programming languages, and may be of predefined finite lengths or of variable lengths. Within the context of the disclosure, the term "character string" may include at least one of a numeral, an alphabetic character, a symbol, a punctuation mark and/or a separator.

According to various embodiments, the term "numeral", as used herein, may refer to a character of or denoting a number.

According to various embodiments, the term "alphabetic character", as used herein, may refer to the conventional 26 characters of the alphabet. In some embodiments, the term "alphabetic character" may further include characters in any other language, e.g. Chinese characters.

According to various embodiments, the term "symbol", as used herein, may refer to conventional symbols as used in the field of ophthalmology, including but not limited to "- (minus)"; "+ (plus)"; "° (degrees)". In various embodiments, the term "symbol" may also refer to the unit of magnifying power of a lens, i.e. diopter (D).

According to various embodiments, the term "punctuation mark" as used herein, may refer to conventional marks as used in the field of ophthalmology, including but not limited to ". (decimal point)"; ", (decimal comma)".

According to various embodiments, the term "separator" as used herein, may refer to one or more spacings (" ") in the optical prescription data. In the disclosure, the separator is denoted as " ".

Reference to the numbering of the first to twentieth character strings, as used herein, are not in sequence or in numerical order. The numberings of the character strings are merely provided for reference and for clarity reasons.

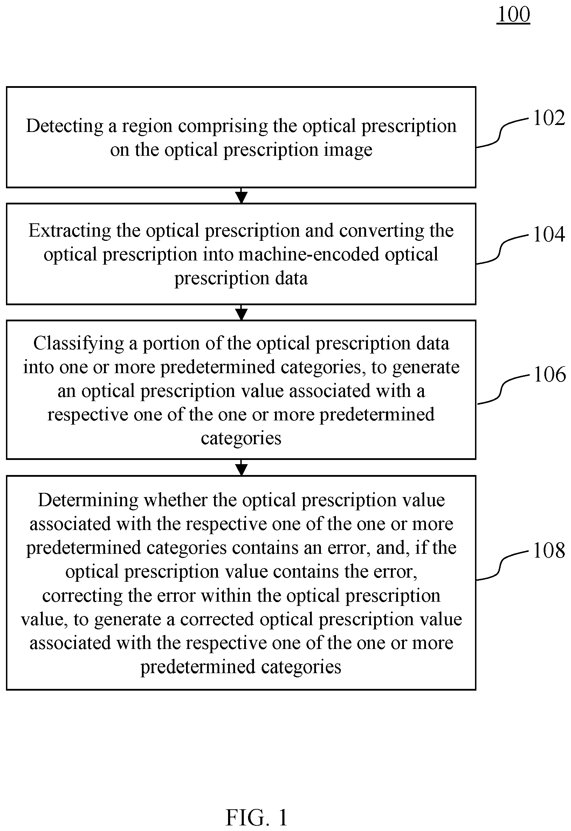

Figure 1 shows a schematic illustration of a method 100 for reading an optical prescription on an optical prescription image. Method 100 includes (i.) detecting a region comprising the optical prescription on the optical prescription image (step 102); (ii.) extracting the optical prescription and converting the optical prescription into machine-encoded optical prescription data (step 104); (iii.) classifying a portion of the optical prescription data into one or more predetermined categories, to generate an optical prescription value associated with a respective one of the one or more predetermined categories (step 106); and (iv.) determining whether the optical prescription value associated with the respective one of the one or more predetermined categories contains an error, and, if the optical prescription value contains the error, correcting the error within the optical prescription value, to generate a corrected optical prescription value associated with the respective one of the one or more predetermined categories (step 108).

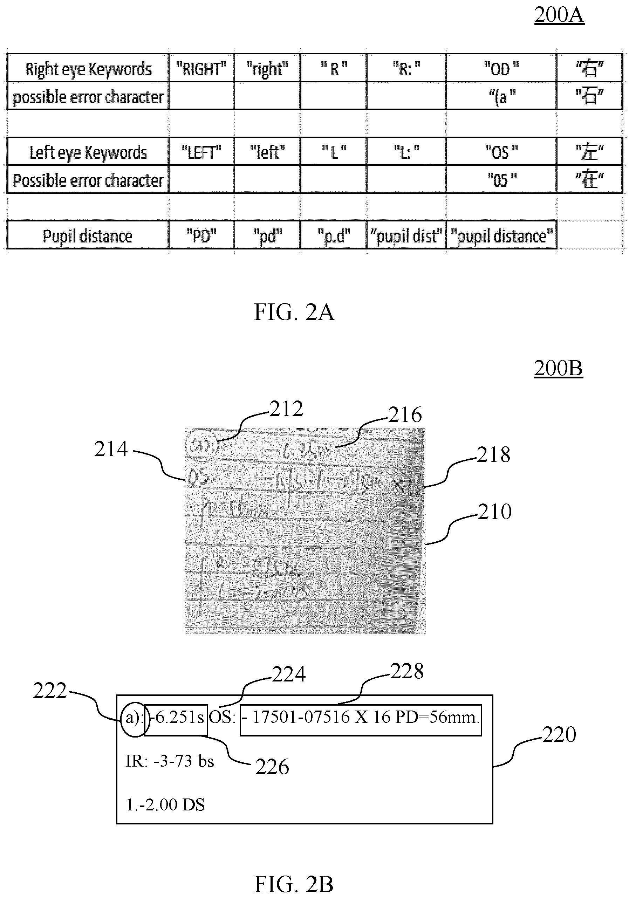

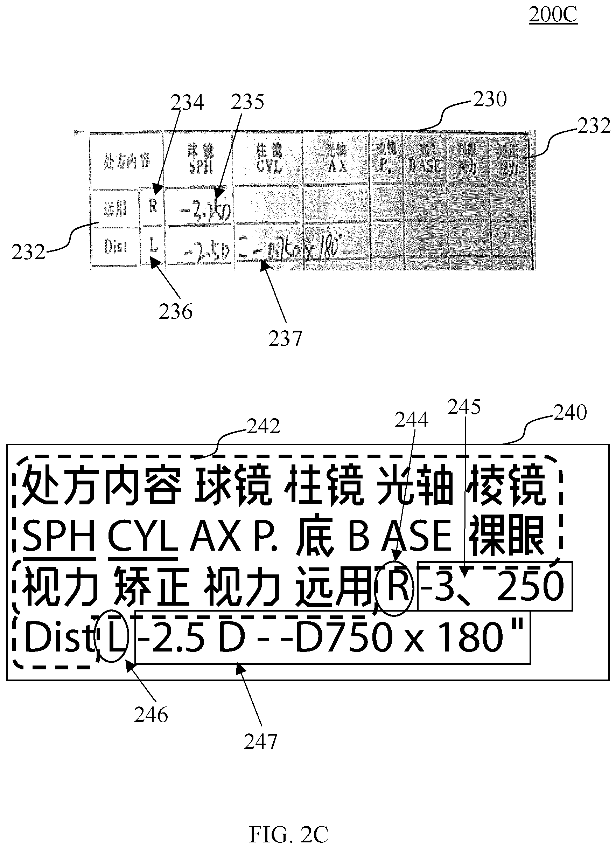

Figure 2A shows an exemplary erroneous keyword table 200A of the at least one keyword and a corresponding erroneous keyword associated with said at least one keyword; figure 2B shows an example 200B of the detection, extraction and conversion ("recording") of a handwritten optical prescription on an optical prescription image 210 into optical prescription data 220; and figure 2C shows another example 200C of the recording of a handwritten optical prescription on a printed prescription form 230 into optical prescription data 240, in accordance with steps 102 and 104 of method 100.

Referring to figure 1 and 2A, detecting a region comprising the optical prescription on the optical prescription image (step 102) may include detecting at least one keyword and a portion of the optical prescription associated with the at least one keyword. Non-limiting keywords denoting the right eye of a subject may include: "RIGHT"; "R "; "R:"; "OD"; " 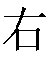 ", and non-limiting keywords denoting the left eye of a subject may include: "LEFT"; "L "; "L:"; "OS"; " 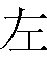 ". Once the at least one keyword is detected, step 102 may include detecting the portion of the optical prescription associated with the at least one keyword, which may be positioned after said keyword. For example, the portion of the optical prescription may be positioned adjacent to and on the RHS of said keyword. Alternatively, or in addition, step 102 may include detecting a first keyword, e.g. "R"; "OD"; " 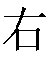 " and a second keyword, e.g. "L"; "OS"; " 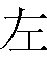 " positioned after the first keyword, and detecting the portion of the optical prescription between the two keywords, which may correspond to the portion of the optical prescription associated with the first keyword, e.g. "R"; "OD"; " 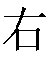 ".

Detecting the at least one keyword in step 102 may further include detecting the at least one keyword and a corresponding one or more erroneous keywords associated with the respective one of the at least one keyword, as shown in erroneous keyword table 200A. For example, the alphabetic characters in keyword "OD" may be detected as a symbol and an alphabetic character, e.g. "(a"; "a)"; and the alphabetic characters in keyword "OS" may be detected as numerals "05".

According to various embodiments, the detection of a corresponding one or more erroneous keywords associated with a respective one of the at least one keyword may be based on a machine learning algorithm. For example, one or more neural networks may be trained by inputting a keyword and the corresponding one or more possible erroneous keywords associated with the keyword, and building a table, e.g. erroneous keyword table 200A or any database containing information that provides the correlation between the keyword and the corresponding one or more erroneous keywords. In an embodiment, said table or database may include training the neural network with a set of optical prescription images, to establish the correlation between the keyword and the corresponding one or more erroneous keyword. The table or database may be stored in a memory or an external server.

Referring to figures 1 to 2C, extracting the optical prescription (step 104) may include extracting the at least one keyword and the portion of the optical prescription associated with the at least one keyword. In some embodiments, the portion of the optical prescription associated with the at least one keyword may be positioned after the keyword. In some other embodiments, the portion of the optical prescription associated with the at least one keyword may be positioned between two keywords. Step 104 also includes extracting the corresponding one or more erroneous keywords associated with the respective one of the at least one keyword that may be detected in accordance with erroneous keyword table 200A, and converting the optical prescription, including the keyword and the portion of the optical prescription associated with the keyword, into machine-encoded optical prescription data. The conversion of the optical prescription into optical prescription data may be performed using electronic means, for example, by digitalizing the printed or handwritten optical prescription using conventional OCR technique, as known to those skilled in the art.

In some embodiments, steps 102 and 104 may further include the detection, extraction and conversion of portions of the optical prescription which may be associated with the subject's general vision condition. The data contained in such portions of the optical prescription image may include general information such as but not limited to: the subject's name, date of birth, date of measurement, measurement type used in the eye examination. It is contemplated that other data relating to the subject's general vision condition may be included in such portions of the optical prescription image.

Referring to the example 200B as shown in figure 2B, the keyword 212 "OD" may be incorrectly recorded as keyword 222 "a)", and the keyword 214 "OS" may be incorrectly recorded as keyword 224 "OS". The corresponding one or more erroneous keywords associated with the keyword may be based on erroneous keyword table 200A.

In an embodiment, the portion of the optical prescription 216 corresponding to keyword 212 "OD" may be recorded as the optical prescription data 226, since it is positioned after, e.g. adjacent and on the RHS of keyword 212 "OD". In another embodiment, the optical prescription 216 positioned between keywords 212, 214, e.g. "OD" and "OS" may be recorded as the optical prescription data 226. Similarly, the portion of the optical prescription 218 corresponding to keyword 214 "OS" may be recorded as optical prescription data 228. For example, the optical prescription 218 may be positioned after keyword 214 "OS" and may therefore be recorded as the optical prescription data 228.

Referring to figure 2C which shows another example 200C including a handwritten optical prescription and printed text of the keywords on the printed prescription form 230, steps 102 and 104 of method 100 may include recording the portion containing the printed keywords 232 (including any Chinese character translation thereof) into optical prescription keyword data 242 on the optical prescription data 240. For example, keyword 234 "R" may be recorded as keyword 244 "R", and the keyword 236 "L" may be recorded as keyword 246 "L". The portion of the optical prescription 235 corresponding to keyword 234 "R" may be recorded as optical prescription data 245, and the portion of the optical prescription 237 corresponding to keyword 236 "L" may be recorded as optical prescription data 247. In various embodiments, the portions of the optical prescriptions 235, 237 which are positioned after its respective keywords 234, 236, e.g. "R", "L" may be recorded as the optical prescription data 245, 247, respectively.

As shown in examples 200B and 200C, the machine-encoded optical prescription data may include or be an incoherent character string of at least one numeral, alphabetic character, symbol, punctuation mark and/or separator, and further includes errors. As such, it may be difficult for the user, e.g. subject or parents of subject, to understand the information presented in the machine-encoded optical prescription data 245, 247, and further classification and/or correction of any errors in the optical prescription data 245, 247 may be required.

Referring back to figure 1, method 100 further includes (iii.) classifying a portion of the machine-encoded optical prescription data into one or more predetermined categories, to generate an optical prescription value associated with a respective one of the one or more predetermined categories (step 106).

The one or more predetermined categories may include (i.) a first category associated with the subject's spherical data, referring to a lens power or spherical power required to correct the vision, e.g. myopia (nearsightedness) or hyperopia (farsightedness) of the subject. The first category may include the lens or spherical power for both the right and left eyes of the subject.

The one or more predetermined categories may further include (ii.) a second category associated with the subject's cylinder data, indicative of a lens power required to correct astigmatism in a subject; and (iii.) a third category associated with the subject's cylinder axis data, indicative of the lens meridian required to correct astigmatism in the subject.

The one or more predetermined categories may further include (iv.) a fourth category associated with the subject's additional lens power, indicative of added magnifying lens power that may be required to correct presbyopia in a subject. The fourth category may include the additional lens power for both the right and left eyes of the subject.

The one or more predetermined categories may further include (v.) a fifth category associated with the subject's pupil distance data, indicative of the distance between the pupils of the two eyes of the subject. In an embodiment, the pupil distance data may be a monocular pupil distance measurement, indicative of a distance between the center of the subject's pupil and the middle of the bridge of the subject's nose, and may include pupil distance data for both the right and left eyes of the subject. In another embodiment, the pupil distance data may be a binocular pupil distance measurement, indicative of a distance between the center of the pupil of one eye and the center of the pupil of the other eye.

The one or more predetermined categories may further include (vi.) a sixth category associated with the subject's axial length, indicative of the combination of the anterior chamber depth, lens thickness and vitreous chamber depth, which may be a contributor to the subject's refractive error.

In some embodiments, the one or more predetermined categories may further include (vii.) a seventh category associated with the subject's general vision condition data. As indicated above, non-limiting examples of the data associated with the seventh category may include or be: the subject's name, date of birth, date of eye examination, type of measurement used in the eye examination, for obtaining the optical prescription for the subject.

According to various embodiments, classifying the portion of the optical prescription data into one or more predetermined categories, to generate the optical prescription value associated with a respective one of the one or more predetermined categories (step 106), may include modifying the optical prescription data such that each portion of the optical prescription data which is to be classified into either of the one or more predetermined categories, for example, either of the first to sixth categories, is separated by a single separator, e.g. " ". For example, a string manipulation or string handling function may be applied to the optical prescription data to remove any additional separators in the optical prescription data. Such string manipulating techniques may be known to those skilled in the art and will not be described in detail in the disclosure.

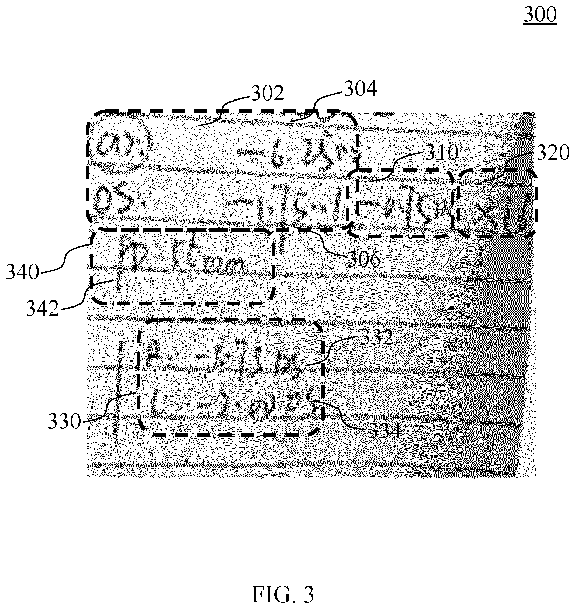

Figure 3 shows an exemplary optical prescription image 300 where each portion of the optical prescription may be positioned in accordance with the general rules and protocols for writing an optical prescription.

As shown in figure 3, the subject's spherical data 302 associated with the first category may be positioned before the data associated with any of the second to sixth categories. For example, the spherical data 302 may be positioned at the top of the optical prescription image 300. The spherical data 302 may include measurements for both the right 304 and left 306 eyes, and in an embodiment, the spherical data for the right 304 eye, e.g. "OD: -6.25" may be positioned before the spherical data for the left 306 eye, e.g. "OS: -1.75". In various embodiments, the spherical data 302 may have the format of: a first part including a symbol such as a "+" or a "-" or no symbol, a second part including 1 to 2 digits, a third part including a punctuation mark such as a "," or a ".", and a fourth part including 2 digits. The spherical data 302 may therefore be 4 to 6 characters in length, e.g. "2.50"; "+2.50"; "+02.25".

The subject's cylinder data 310 associated with the second category may be positioned after, e.g. adjacent to and on the RHS of, the subject's spherical data 302, as shown in figure 3. In an embodiment, the cylinder data 310 may be positioned after the left 306 eye spherical data, e.g. "-0.75" positioned after the left 306 eye spherical data of "-1.75". Alternatively, the cylinder data 310 may be positioned after the right 304 eye spherical data. In various embodiments, the cylinder data 310 may have the format of: a first part including a symbol such as a "+" or a "-" or no symbol, a second part which including 1 to 2 digits, a third part including a punctuation mark such as a "," or a ".", and a fourth part including 2 digits. The cylinder data 310 may therefore be 4 to 6 characters in length, e.g. "0.75"; " -0.75 "; "-4.00".

The subject's cylinder axis data 320 associated with the third category may be positioned after, e.g. adjacent to and on the RHS of, the subject's cylinder data 310, as shown in figure 3. In various embodiments, the subject's cylinder axis data 320 may have the format of: a series of characters and may not include a symbol and/or punctuation mark. In some embodiments, the cylinder axis data 320 may be an integer, and may be 1 to 3 characters in length, e.g. "6"; "16"; "180". In some other embodiments, the cylinder axis data 320 may include the cylinder axis data keyword, such as "A"; "x": "Axis" positioned before the cylinder axis data 320, e.g. "x 6"; "x 16"; "x 180", and may be 3 to 5 characters in length.

The subject's additional lens power data 330 associated with the fourth category may be positioned after the subject's spherical data 302, cylinder data 310, and cylinder axis data 320. As shown in figure 3, the additional lens power data 330 may be positioned in the rows following the spherical data 302, cylinder data 310, and cylinder axis data 320. The additional lens power data 330 may include measurements for the right 332 and/or left 334 eyes, and in an embodiment, the additional lens power data 330 for the right 332 eye, e.g. "R: -3.75" may be positioned before, e.g. in the row preceding the additional lens power data 330 for the left 334 eye, e.g. "L: -2.00". In various embodiments, the additional lens power data 330 may have a same format as the spherical data 302, specifically: a first part including a symbol such as a "+" or a "-" or no symbol, a second part including 1 to 2 digits, a third part including a punctuation mark such as a "," or a ".", and a fourth part including 2 digits. The additional lens power data 330 may therefore be 4 to 6 characters in length, e.g. "3.50"; "+3.50"; "- 2.00".

The subject's pupil distance data 340 associated with the fifth category may be positioned after the keyword 342 associated with the pupil distance data 340, e.g. "PD". Non-limiting examples of keywords 342 associated with pupil distance data 340 may include keywords such as "PD" or "p.d.". In the embodiment as shown in figure 3, the binocular pupil distance data 340 may be positioned in the row following the spherical data 302, cylinder data 310 and cylinder axis data 320, and may be positioned after the keyword 342 "PD". In various embodiments, the pupil distance data 340 may have the format of: a series of characters and may not include a symbol and/or a punctuation mark. In some embodiments, the pupil distance data 340 may be an integer comprising a length of 2 characters, e.g. "25"; "50"; "72". In some embodiments, the pupil distance data 340 may include the unit of measurement, e.g. mm (millimeters) positioned after the pupil distance data 340, and the pupil distance data 340 may be 4 characters in length. While figure 3 shows a binocular pupil distance data 340 measurement, the pupil distance data 340 may be monocular measurements, and may include the pupil distance data 340 for the right and left eyes of the subject.

The subject's axial length data associated with the sixth category may be positioned after the keyword associated with the axial length data (not shown in figure 3). Non-limiting examples of keywords associated with axial length data may include keywords such as "AL" or "a.l.". The axial length data may be a separate measurement and may be positioned in a row separate to and following the rows having the spherical data 302, cylinder data 310, cylinder axis data 320, the additional lens power data 330, and the pupil distance data 340. In various embodiments, the axial length data may have a format of: a first part including 2 digits, a second part including a punctuation mark such as a "," or a ".", and a third part including 2 digits, and may therefore be 5 characters in length, e.g. "20.00"; "27.00". In some embodiments, the axial length data may include the unit of measurement, e.g. mm (millimeters) positioned after the axial length data. As such, the axial length data may be 7 characters in length, e.g. "20.00mm"; "27.00mm".

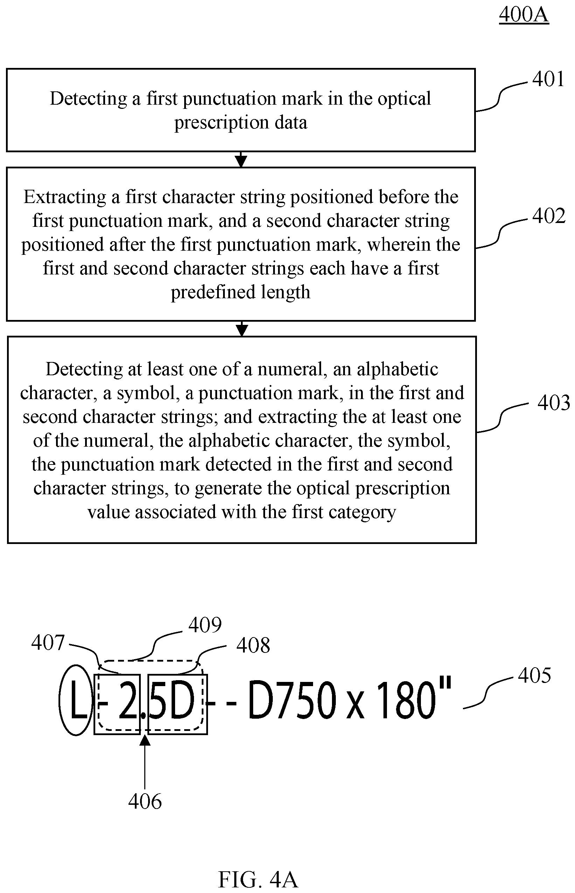

The various embodiments for classifying the portion of the optical prescription data into one or more predetermined categories, to generate the optical prescription value associated with a respective one of the one or more predetermined categories (step 106), will be described with reference to figures 4A to 6D.

Figures 4A to 4D show exemplary schematic illustrations of methods 400A, 400B, 400C, 400D for classifying the portion of the optical prescription data into one or more predetermined categories, in accordance with an embodiment of the disclosure.

Figure 4A shows a flowchart of method 400A for classifying the portion of the optical prescription data into the first category associated with the subject's spherical data, including an example 405 of the same. Method 400A may include (i.) detecting a first punctuation mark in the optical prescription data (step 401), for example, a "," or a "." in the optical prescription data; and (ii.) extracting a first character string positioned before the first punctuation mark, and a second character string positioned after the first punctuation mark, wherein the first and second character string each have a first predefined length (step 402). In some embodiments, the first predefined length may be 3 characters in length. As indicated above, the spherical data may be 4 to 6 characters in length, and may have the format as specified above. Accordingly, the 3 characters, e.g. first character string, positioned before the first punctuation mark, and the 3 characters, e.g. second character string, positioned after the first punctuation mark may be extracted. As shown in example 405, the first character string 407 may be the 3 characters "- 2" positioned before the first punctuation mark 406, and the second character string 408 may be the 3 characters "5D " positioned after the first punctuation mark 406. It is contemplated that the first punctuation mark 406 may also be extracted in step 402.

In some embodiments, method 400A may further include (iii.) detecting at least one of a numeral, an alphabetic character, a symbol and/or a punctuation mark in the first and second character strings, and extracting the least one of the numeral, the alphabetic character, the symbol and/or the punctuation mark in the first and second character strings, to generate the optical prescription value associated with the first category (step 403). Since the spherical data which includes 4 to 6 characters may be contained within the first and second character strings, the extracted one of the numeral, the alphabetic character, the symbol and/or the punctuation mark in the first and second character strings, may correspond to the optical prescription value for the spherical data associated with the first category, and may have a length of less than or equal to 5 characters. As shown in example 405, the optical prescription value 409 of "- 25D" may refer to the optical prescription value 409 for the spherical data associated with the first category.

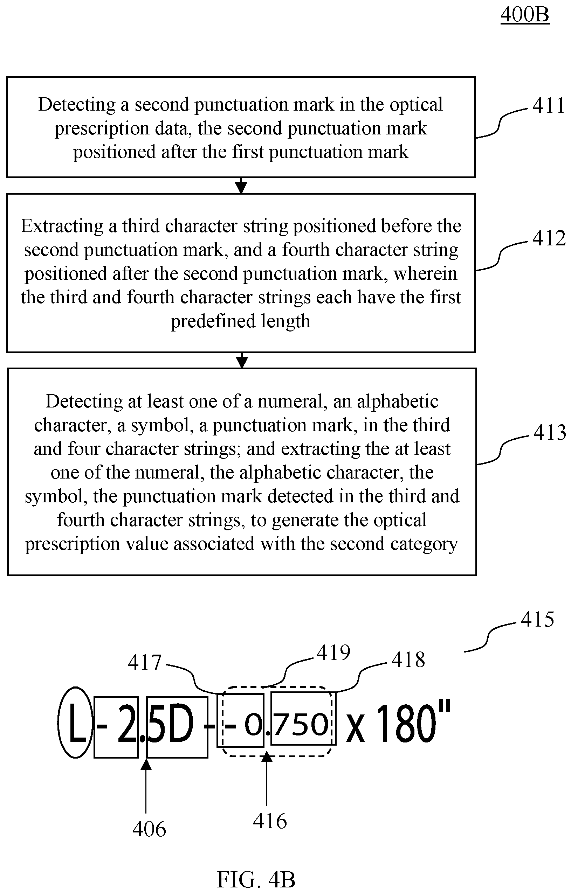

Figure 4B shows a flowchart of method 400B for classifying the portion of the optical prescription data into the second category associated with the subject's cylinder data, including an example 415 of the same. Method 400B may include (i.) detecting a second punctuation mark in the optical prescription data, the second punctuation mark positioned after the first punctuation mark (step 411). In example 415, the second and subsequent punctuation mark 416, e.g. ".", positioned after the first punctuation mark 406 may be detected. Method 400B may also include (ii.) extracting a third character string positioned before the second punctuation mark, and a fourth character string positioned after the second punctuation mark, wherein the third and fourth character string each have the first predefined length (step 412) of 3 characters. In some embodiments, method 400A may be performed before method 400B, since the cylinder data may be positioned after the spherical data. In addition, as indicated above, the cylinder data may be 4 to 6 characters in length, and may have the format as specified above. Accordingly, step 412 of method 400B may include extracting the 3 characters, positioned before and after second punctuation mark, e.g. third and fourth character strings. In example 415, the third character string 417 may be the 3 characters "- 0", and the fourth character string 418 may be the 3 characters "750" positioned before and after the second punctuation mark 416, respectively. It is contemplated that step 412 may further include extraction of the second punctuation mark 416.

In some embodiments, method 400B may further include (iii.) detecting at least one of a numeral, an alphabetic character, a symbol and/or a punctuation mark in the third and fourth character strings, and extracting the same in the third and fourth character strings, to generate the optical prescription value associated with the second category (step 413). Since the cylinder data which includes 4 to 6 characters may be contained within the third and fourth character strings, the extracted one of the numeral, the alphabetic character, the symbol and/or the punctuation mark in the third and fourth character strings, may correspond to the optical prescription value for the cylinder data associated with the second category, and may have a length of less than or equal to 5 characters. As shown in example 415, the optical prescription value 419 of "-0750" may refer to the optical prescription value 419 for the cylinder data associated with the second category.

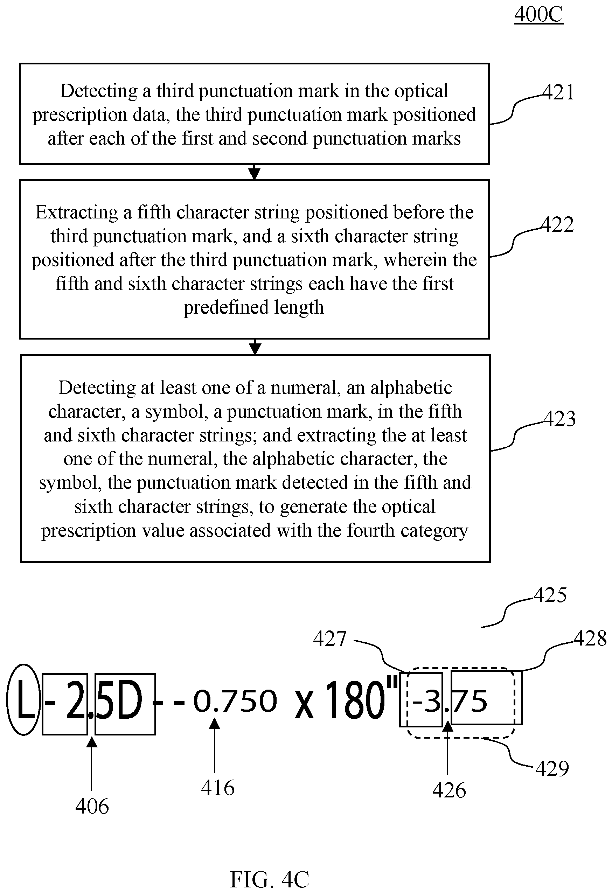

Figure 4C shows a flowchart of method 400C for classifying the portion of the optical prescription data into the fourth category associated with the subject's additional lens power data, including an example 425 of the same. Method 400C may include (i.) detecting a third punctuation mark in the optical prescription data, the third punctuation mark positioned after each of the first and second punctuation marks (step 421). As indicated above, the additional lens power data may be a separate measurement and may be positioned after the spherical data (having the first punctuation mark) and the cylinder data (having the second punctuation mark). For example, the third punctuation mark 426, e.g. ".", may be positioned after the first 406 and second 416 punctuation marks. Method 400C may further include (ii.) extracting a fifth character string positioned before the third punctuation mark, and a sixth character string positioned after the third punctuation mark, wherein the fifth and sixth character strings each have the first predefined length (step 422) of 3 characters. The additional lens power data may have a length of 4 to 6 characters, and the format as explained above. Accordingly, methods 400A and 400B may be performed before performing method 400C, and the 3 characters positioned before, e.g. fifth character string, and after, e.g. sixth character string the third punctuation mark may be extracted. As shown in example 425, the 3 characters positioned before the third punctuation mark 426, and corresponding to the fifth character string 427 may be " -3", and the 3 characters positioned after the third punctuation mark 426 and corresponding to the sixth character string 428, e.g. "75 ", may be extracted. It is contemplated that step 422 may further include extraction of the third punctuation mark 426.

In some embodiments, method 400C may further include (iii.) detecting at least one of a numeral, an alphabetic character, a symbol and/or a punctuation mark in the fifth and sixth character strings, and extracting the same to generate the optical prescription value associated with the fourth category (step 423). Since the additional lens power data which includes 4 to 6 characters may be contained within the fifth and sixth character strings, the extracted one of the numeral, the alphabetic character, the symbol and/or the punctuation mark in the fifth and sixth character strings, may correspond to the optical prescription value for the additional lens power data of the fourth category. In an embodiment, the optical prescription value for the additional lens data may have a length of less than or equal to 5 characters. As shown in example 425, the optical prescription value 429 of "-375 " may refer to the optical prescription value 429 for the additional lens data associated with the fourth category.

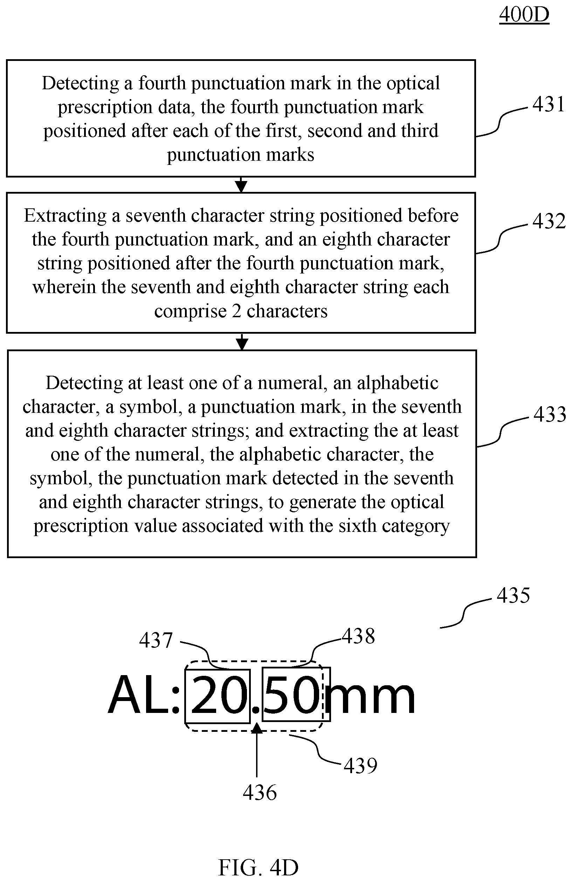

Figure 4D shows a flowchart of method 400D for classifying the portion of the optical prescription data into the sixth category associated with the subject's axial length data, including an example 435 of the same. Method 400D may include (i.) detecting a fourth punctuation mark in the optical prescription data, the fourth punctuation mark positioned after each of the first, second and third punctuation marks (step 431). In example 445, the fourth punctuation mark 436, e.g. the subsequent "." with respect to the first 406, second 416 and third 426 punctuation marks associated with the first to sixth character strings (see figures 4A to 4C), may be detected. Method 400D may further include (ii.) extracting a seventh character string positioned before the fourth punctuation mark, and an eighth character string positioned after the fourth punctuation mark, wherein the seventh and eighth character string each comprise 2 characters (step 432). As mentioned above, the axial length data may be a separate measurement and have a length of 5 (excluding units of measurement), or 7 characters (including units of measurement), and the format as described above. Accordingly, and as shown in example 435, the 2 characters positioned before and corresponding to the seventh character string 437, e.g. "20" the fourth punctuation mark 436, and after and corresponding to the eighth character string 438, e.g. "50" the fourth punctuation mark 436, may be extracted. It is contemplated that step 432 may further include extraction of the fourth punctuation mark 436. In some embodiments, methods 400A to 400C may be performed before performing method 400D.

In some embodiments, method 400D may further include (iii.) detecting at least one of a numeral, an alphabetic character, a symbol and/or a punctuation mark in the seventh and eighth character strings, and extracting the same to generate the optical prescription value associated with the sixth category (step 433). Since the axial length data which includes 5 to 7 characters may be contained within the seventh and eighth character strings, the extracted one of the numeral, the alphabetic character, the symbol and/or the punctuation mark in the seventh and eighth character strings, may correspond to the optical prescription value for the axial length data of the sixth category. In an embodiment, the optical prescription value of the axial length data may have a length of less than or equal to 5 characters, and may have the optical prescription value 439 of "2050" as shown in example 435.

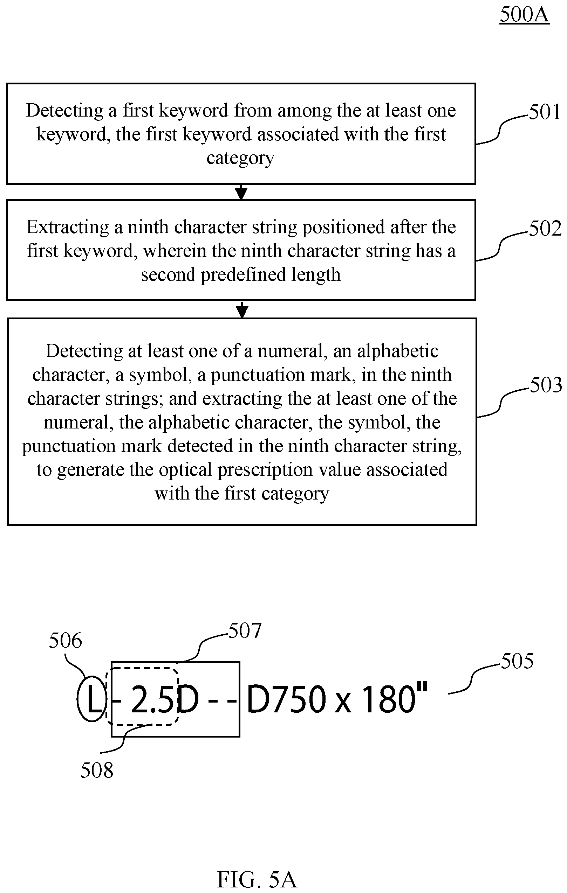

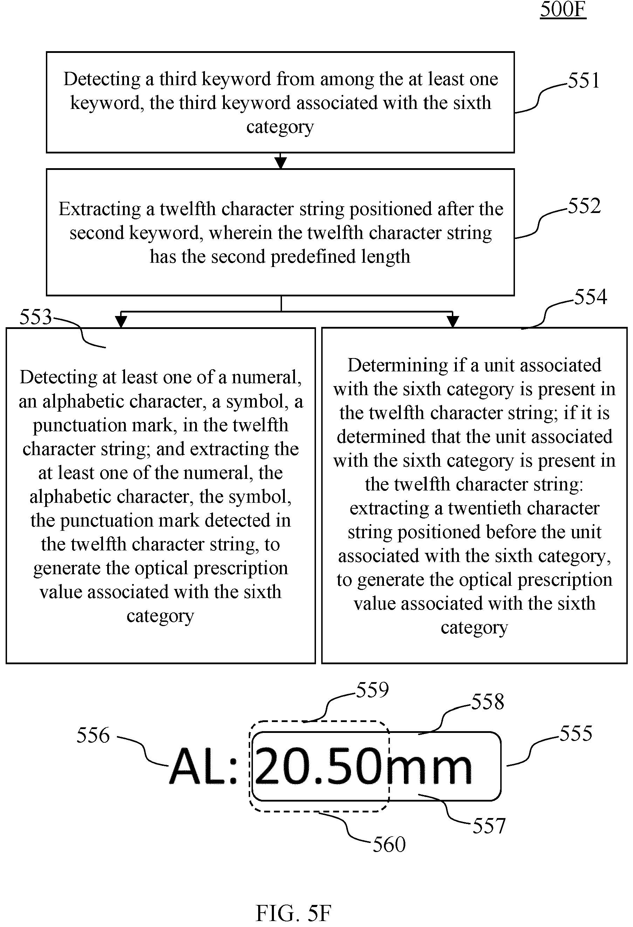

Figures 5A to 5F show exemplary schematic illustrations of methods 500A, 500B, 500C, 500D, 500E, 500F for classifying the portion of the optical prescription data into one or more predetermined categories, in accordance with another embodiment of the disclosure.

Figure 5A shows a flowchart of method 500A for classifying the portion of the optical prescription data into the first category associated with the subject's spherical data, including an example 505 of the same. Method 500A may include (i.) detecting a first keyword from among the at least one keyword, the first keyword associated with the first category (step 501). For example, the keyword "L" 506 associated with the spherical data may be detected in example 505. Method 500A may further include (ii.) extracting a ninth character string positioned after the first keyword, wherein the ninth character string has a second predefined length (step 502). In some embodiments, the second predefined length may include or be 7 characters in length. As indicated above, the spherical data may include 4 to 6 characters, and may be contained within the 7 characters of the ninth character string. As shown in example 505, the ninth character string 507 which is positioned after the keyword 506 and comprises 7 characters, e.g. "-2.5D--" may be extracted.

In some embodiments, method 500A may further include (iii.) detecting at least one of a numeral, an alphabetic character, a symbol and/or a punctuation mark in the ninth character string, and extracting the least one of the numeral, the alphabetic character, the symbol and/or the punctuation mark in the ninth character string, to generate the optical prescription value associated with the first category (step 503). Since the spherical data comprising 4 to 6 characters may be contained within the ninth character string, the extracted one of the numeral, the alphabetic character, the symbol and/or the punctuation mark in the ninth character string, may correspond to the optical prescription value for the spherical data of the first category, and may have a length of less than or equal to 5 characters. For example, the optical prescription value 508 of "- 2.5" may refer to the optical prescription value 508 for the spherical data of the first category.

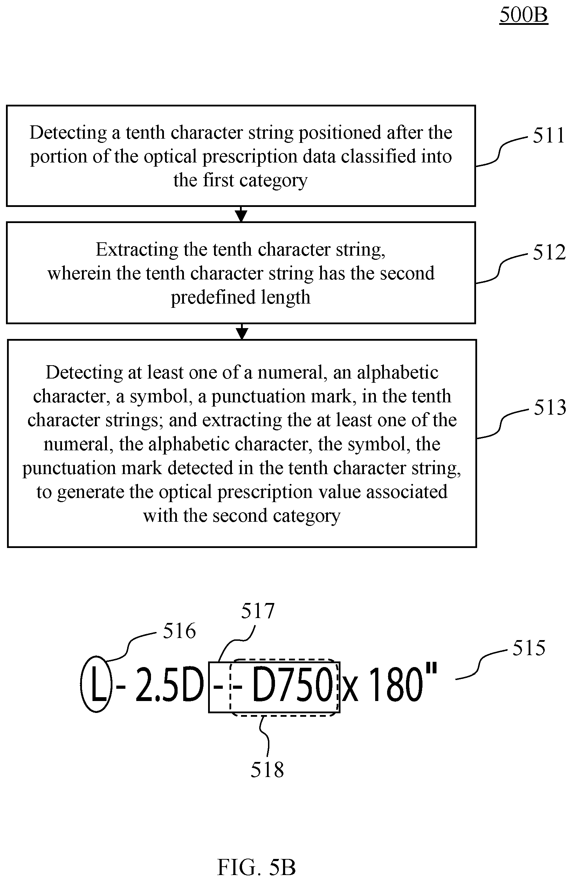

Figure 5B shows a flowchart of method 500B for classifying the portion of the optical prescription data into the second category associated with the subject's cylinder data, including an example 515 of the same. Method 500B includes (i.) detecting a tenth character string positioned after the portion of the optical prescription data classified into the first category (step 511); and (ii.) extracting the tenth character string, wherein the tenth character string has the second predefined length (step 512) of 7 characters. Since the cylinder data may be positioned after the spherical data, for example, spherical data for either the left or right eye, method 500B may include the detection and extraction of the tenth character string comprising the 7 characters positioned after the spherical data. The cylinder data which comprises 4 to 6 characters in length may be contained within the 7 characters of the tenth character string. As shown in example 515, the tenth character string 517, e.g. "-- D750" may be positioned after the spherical data 516. In some embodiments, method 500A may be performed before method 500B.

In some embodiments, method 500B may further include (iii.) detecting at least one of a numeral, an alphabetic character, a symbol and/or a punctuation mark in the tenth character string, and extracting the same in the tenth character string, to generate the optical prescription value associated with the second category (step 513). Since the cylinder data which includes 4 to 6 characters may be contained within the tenth character string, the extracted one of the numeral, the alphabetic character, the symbol and/or the punctuation mark in the tenth character string, may correspond to the optical prescription value for the cylinder data of the second category. In an embodiment, the optical prescription value of the cylinder data may have a length of less than or equal to 5 characters, and may have the optical prescription value 518 of "-D750", as shown in example 515.

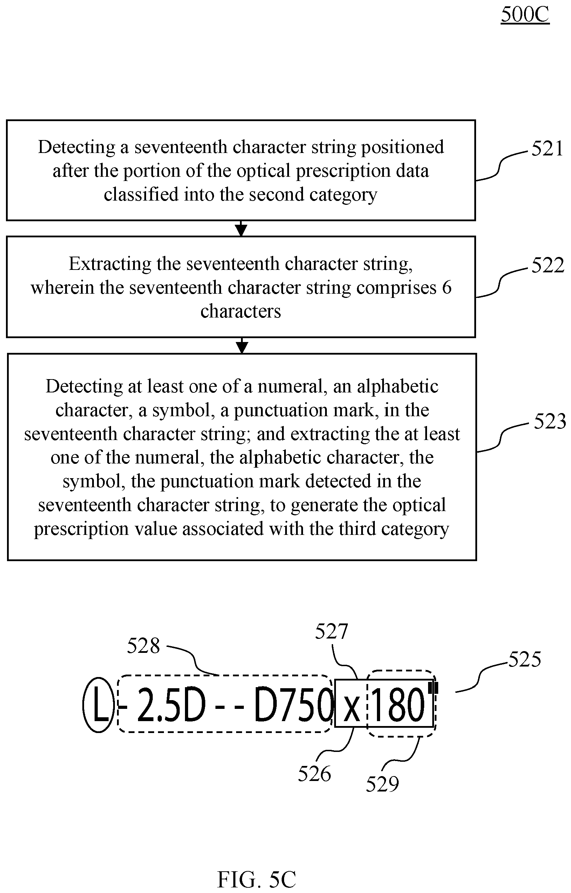

Figure 5C shows a flowchart of method 500C for classifying the portion of the optical prescription data into the third category associated with the subject's cylinder axis data, including an example 525 of the same. Method 500C includes (i.) detecting a seventeenth character string positioned after the portion of the optical prescription data classified into the second category (step 521); and (ii.) extracting the seventeenth character string, wherein the seventeenth character string comprises 6 characters (step 522). As indicated above, the cylinder axis data may be positioned after the cylinder data (since the cylinder axis data may be usually measured in conjunction with the cylinder data), and accordingly, method 500C may include the detection and extraction of the seventeenth character string comprising the 6 characters positioned after the cylinder data. The cylinder axis data which comprises 1 to 3 characters (excluding keyword 526 "x"), or 2 to 4 characters (including keyword 526 "x") in length may be contained within the 6 characters of the seventeenth character string. As shown in example 525, the seventeenth character string 527 having a length 6 characters, e.g. " x 180" may be positioned after the spherical data and cylinder data 528. In some embodiments, the spherical and cylinder data 528 may be detected prior to detecting the cylinder axis data 527, and methods 500A and 500B may be performed before method 500C.

In some embodiments, method 500C may further include (iii.) detecting at least one of a numeral, an alphabetic character, a symbol and/or a punctuation mark in the seventeenth character string, and extracting the same in the seventeenth character string, to generate the optical prescription value associated with the third category (step 523). Since the cylinder axis data which includes 1 to 4 characters may be contained within the seventeenth character string, the extracted one of the numeral, the alphabetic character, the symbol and/or the punctuation mark in the seventeenth character string, may correspond to the optical prescription value for the cylinder axis data of the third category. In an embodiment, the optical prescription value of the cylinder axis data may have a length of less than or equal to 4 characters, and may have the optical prescription value 529 of "180" as shown in example 525.

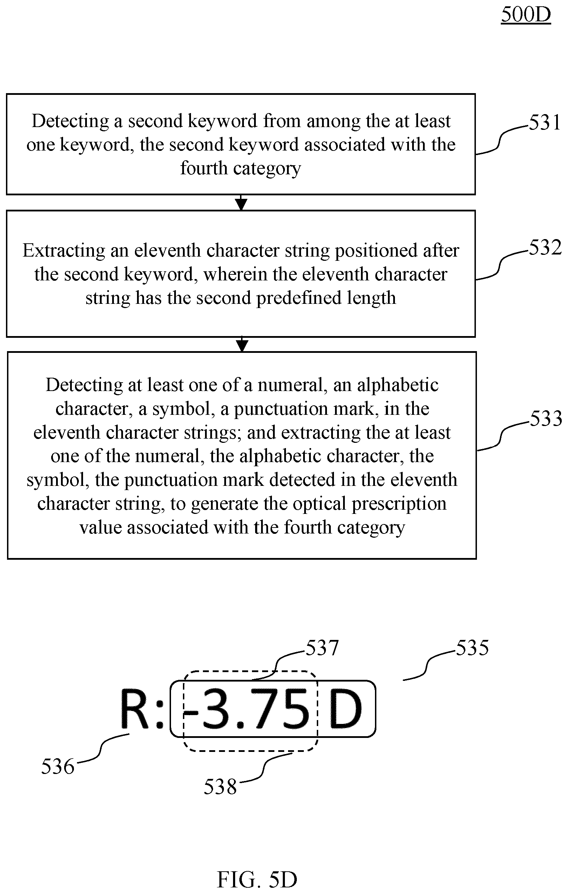

Figure 5D shows a flowchart of method 500D for classifying the portion of the optical prescription data into the fourth category associated with the subject's additional lens power data, including an example 535 of the same. Method 500C includes (i.) detecting a second keyword from among the at least one keyword, the second keyword associated with the fourth category (step 531). For example, the keyword 536 "R" associated with the subject's additional lens power data may be detected in example 535. Method 500C further includes (ii.) extracting an eleventh character string positioned after the second keyword, wherein the eleventh character string has the second predefined length (step 532), which may be 7 characters in length. Since the additional lens power data may include 4 to 6 characters, said data may be contained within the 7 characters of the eleventh character string. As shown in example 535, the eleventh character string 537 which is positioned after the keyword 536 "R" and including 7 characters, e.g. "-3.75 D" may be extracted.

In some embodiments, method 500C may further include (iii.) detecting at least one of a numeral, an alphabetic character, a symbol and/or a punctuation mark in the eleventh character string, and extracting the same in the eleventh character string, to generate the optical prescription value associated with the fourth category (step 533). Since the additional lens power data which includes 4 to 6 characters may be contained within the eleventh character string, the extracted one of the numeral, the alphabetic character, the symbol and/or the punctuation mark in the eleventh character string, may correspond to the optical prescription value for the additional lens power data of the fourth category. In an embodiment, the optical prescription value of the additional lens power data may have a length of less than or equal to 5 characters, and may have the optical prescription value 538 of "-3.75" as shown in example 535.

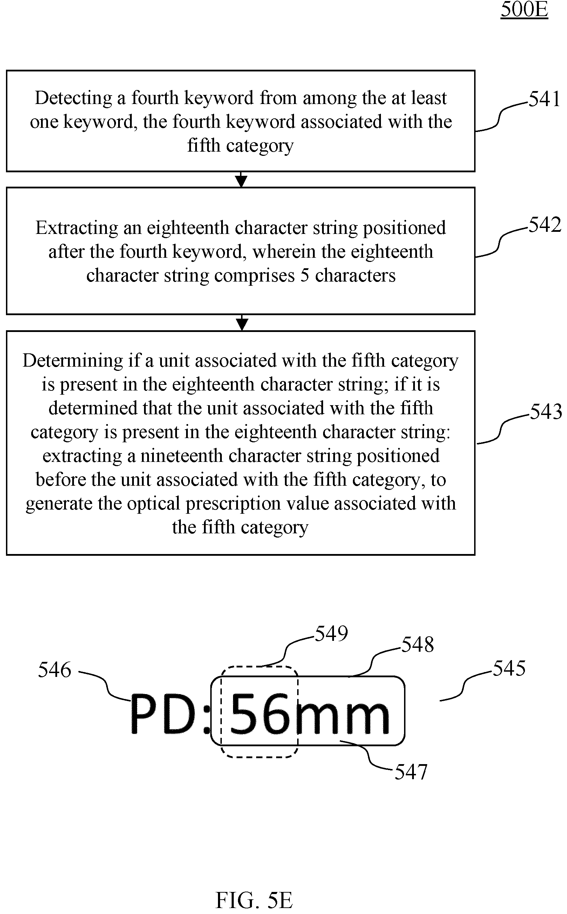

Figure 5E shows a flowchart of method 500E for classifying the portion of the optical prescription data into the fifth category associated with the subject's pupil distance data, including an example 545 of the same. Method 500E may include (i.) detecting a fourth keyword from among the at least one keyword, the fourth keyword associated with the fifth category (step 541). For example, the keyword 546 "PD" associated with the subject's pupil distance data may be detected in example 545. Method 500E may further include (ii.) extracting an eighteenth character string positioned after the fourth keyword, wherein the eighteenth character string comprises 5 characters in length (step 542). The pupil distance axis data which comprises 2 characters (excluding the unit associated with the fifth category 547 "mm"), or 4 characters (including unit associated with the fifth category 547 "mm") in length may be contained within the 5 characters of the eighteenth character string. As shown in example 545, the eighteenth character string 548 having a length 5 characters, e.g. " 56mm", and which is positioned after the keyword 546 "PD", may be extracted. In some embodiments, the spherical, cylinder, cylinder axis data and additional lens power data may be detected prior to detecting the pupil distance data 548, and the methods of the present disclosure may include performing method 500A to 500D before method 500E.

In some embodiments, method 500E may further include (iii.) determining if a unit associated with the fifth category is present in the eighteenth character string; if it is determined that the unit associated with the fifth category is present in the eighteenth character string: extracting a nineteenth character string positioned before the unit associated with the fifth category, to generate the optical prescription value associated with the fifth category (step 543). In an embodiment, step 543 may include detecting the unit associated with the fifth category 547, e.g. "mm", and extracting the nineteenth character string 549 positioned before the unit associated with the fifth category 547, e.g. "56", which may have a length less than or equal to 2 characters. In another embodiment, if it is determined that the unit associated with the fifth category 547 is not present in the eighteenth character string 548, step 543 may include extracting the 2 characters positioned after the keyword 546 "PD", e.g. "56", to generate the optical prescription value associated with the fifth category.

Figure 500F shows a flowchart of method 500F for classifying the portion of the optical prescription data into the sixth category associated with the subject's axial length data, including an example 555 of the same. Method 500F may include (i.) detecting a third keyword from among the at least one keyword, the third keyword associated with the sixth category (step 551). For example, the keyword 556 "AL" associated with the subject's axial length may be detected in example 555. Method 500F may further include (ii.) extracting a twelfth character string positioned after the third keyword, wherein the twelfth character string has the second predefined length (step 552), comprising 7 characters. The axial length data which comprises 5 characters (excluding the unit associated with the sixth category 557 "mm") may be contained within the 7 characters of the twelfth character string. As shown in example 555, the twelfth character string 558 having a length 7 characters, e.g. "20.50mm" may be positioned after the keyword 556 "AL", and may be extracted in accordance with steps 551 and 552 of method 500F. In some embodiments, the spherical, cylinder, cylinder axis data, additional lens power data and pupil distance data may be detected prior to detecting the axial length data 558, and the methods of the present disclosure may include performing method 500A to 500E before method 500F.

In some embodiments, method 500F may further include (iii.) detecting at least one of a numeral, an alphabetic character, a symbol and/or a punctuation mark in the twelfth character string, and extracting the same in the twelfth character string, to generate the optical prescription value associated with the sixth category (step 553). Since the axial length data which includes 5 characters may be contained within the twelfth character string, the extracted one of the numeral, the alphabetic character, the symbol and/or the punctuation mark in the twelfth character string, may correspond to the optical prescription value for the axial length data of the sixth category. In an embodiment, the optical prescription value of the axial length data may have a length of less than or equal to 5 characters, and may have the optical prescription value 559 of "20.50" as shown in example 555.

Alternatively, in some other embodiments, method 500F may include (iii.) determining if a unit associated with the sixth category is present in the twelfth character string; if it is determined that the unit associated with the sixth category is present in the twelfth character string: extracting a twentieth character string positioned before the unit associated with the sixth category, to generate the optical prescription value associated with the sixth category (step 554). In an embodiment, step 554 may include detecting the unit associated with the sixth category 557, e.g. unit "mm", and extracting the twentieth character string 560 positioned before the unit associated with the sixth category 557, e.g. "20.50", which may have a length less than or equal to 5 characters. In another embodiment, if it is determined that the unit associated with the sixth category 557 is not present in the twelfth character string 558, step 554 may include extracting the 5 characters positioned after the keyword 556 "AL", e.g. "20.50", to generate the optical prescription value associated with the sixth category.

According to various embodiments, it is contemplated that the methods of the present disclosure may be implemented for classifying the portion of the optical prescription data into the seventh category associated with the subject's general vision condition, to generate the optical prescription value associated with the seventh category. For example, a fifth keyword associated with the subject's general vision condition, from among the at least one keywords may be detected and a character string positioned after the fifth keywords may be extracted. Non-limiting examples of the fifth keywords include: "Name"; "Date of measurement"; "Solution type"; and the character string positioned after the fifth keyword may have a variable length. In an embodiment, generating the optical prescription value associated with the seventh category may further include detecting at least one of a numeral, an alphabetic character, a symbol and/or a punctuation mark in said character string, and extracting the same, since the general vision condition data may be contained within the character string having a variable length.

According to various embodiments, detecting the first to fifth keywords associated with the respective one of the one or more predetermined categories, may include detecting a corresponding one or more erroneous keywords associated with the respective one of the at least one keyword. For example, the detection of the erroneous keywords may be based on erroneous keyword table 200A.

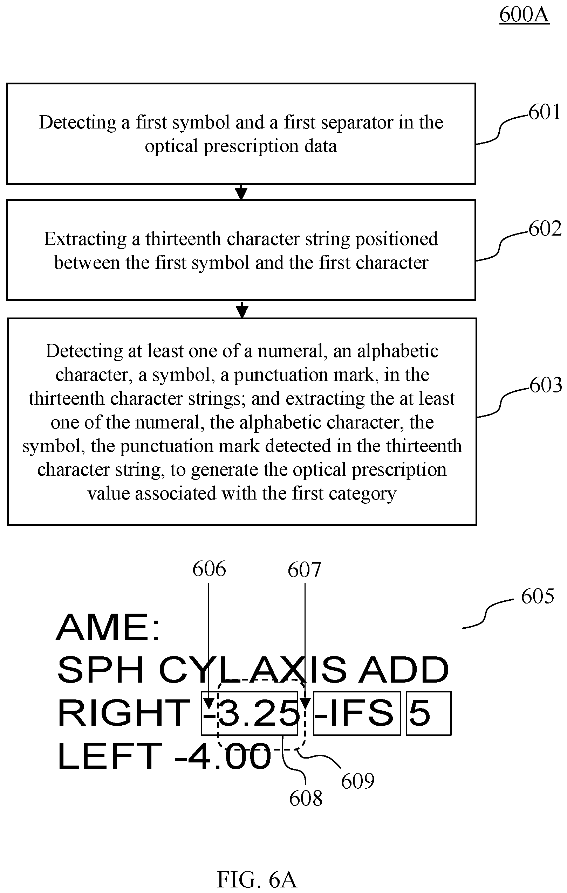

Figures 6A to 6D show exemplary schematic illustrations of methods 600A, 600B, 600C, 600D for classifying the portion of the optical prescription data into one or more predetermined categories, in accordance with another embodiment of the disclosure.

Figure 6A shows a flowchart of method 600A for classifying the portion of the optical prescription data into the first category associated with the subject's spherical data, including an example 605 of the same. Method 600A may include (i.) detecting a first symbol and a first separator in the optical prescription data (step 601); and (ii.) extracting a thirteenth character string positioned between the first symbol and the first separator (step 602). As mentioned above, the spherical data is typically the first measurement positioned on the optical prescription image. For example, the spherical data may be positioned before the cylinder data, and may be separated by a single separator, e.g. " ". In this embodiment, the optical prescription data may be manipulated to remove additional separators prior to step 601. Accordingly, and as shown in example 605, step 601 may include detecting the first symbol 606 and the first separator 607, and extracting the thirteenth character string 608, e.g. "3.25" positioned between the first symbol and the first separator. It is contemplated that embodiments, step 602 may further include extracting the first symbol 606 and the first separator 607.

In some embodiments, method 600A may further include (iii.) detecting at least one of a numeral, an alphabetic character, a symbol and/or a punctuation mark in the thirteenth character string, and extracting the least one of the numeral, the alphabetic character, the symbol and/or the punctuation mark in the thirteenth character string, to generate the optical prescription value associated with the first category (step 603). Since the spherical data which include 4 to 6 characters may be contained within the thirteenth character string, the extracted one of the numeral, the alphabetic character, the symbol and/or the punctuation mark in the thirteenth character string, may correspond to the optical prescription value for the spherical data of the first category. In an embodiment, the optical prescription value of the spherical data may have a length of less than or equal to 5 characters. As shown in example 605, the optical prescription value 609, e.g. "3.25"; or "-3.25", may be extracted.

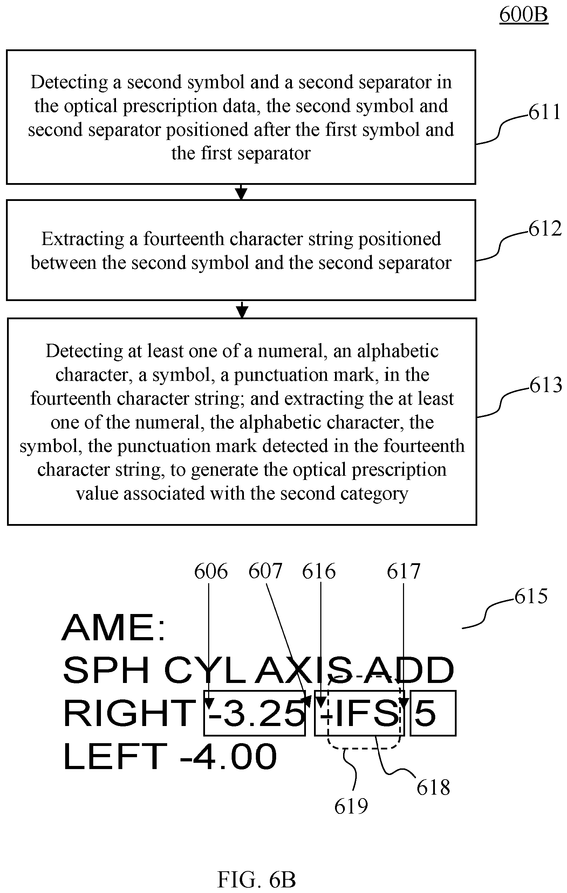

Figure 6B shows a flowchart of method 600B for classifying the portion of the optical prescription data into the second category associated with the subject's cylinder data, including an example 615 of the same. Method 600B may include (i.) detecting a second symbol and a second separator in the optical prescription data, the second symbol and second separator positioned after the first symbol and the first separator (step 611); and (ii.) extracting a fourteenth character string positioned between the second symbol and the second separator (step 612). As mentioned above, cylinder data is typically positioned after the spherical data on the optical prescription image. For example, the cylinder data may be positioned after the spherical data, and may be separated by a single separator " " (via a string manipulation function as described above). Accordingly, method 600A may be performed prior to method 600B. As shown in example 615, step 611 may thus include detecting the second symbol 616 and the second separator 617, which are positioned after the first symbol 606 and first separator 607, and extracting the fourteenth character string 618, e.g. "IFS" positioned between the second symbol 616 and the second separator 617. It is contemplated that step 611 may further include extracting the second symbol 616 and the second separator 617.

In some embodiments, method 600B may further include (iii.) detecting at least one of a numeral, an alphabetic character, a symbol and/or a punctuation mark in the fourteenth character string, and extracting the same in the fourteenth character string, to generate the optical prescription value associated with the second category (step 613). As mentioned above, the cylinder data which may include 4 to 6 characters may be contained within the fourteenth string, the extracted one of the numeral, the alphabetic character, the symbol and/or the punctuation mark in the fourteenth character string, may correspond to the optical prescription value for the cylinder data of the second category. In an embodiment, the optical prescription value of the cylinder data may have a length of less than or equal to 5 characters, and may have the optical prescription value 619 of "IFS"; or "-IFS", as shown in example 615.

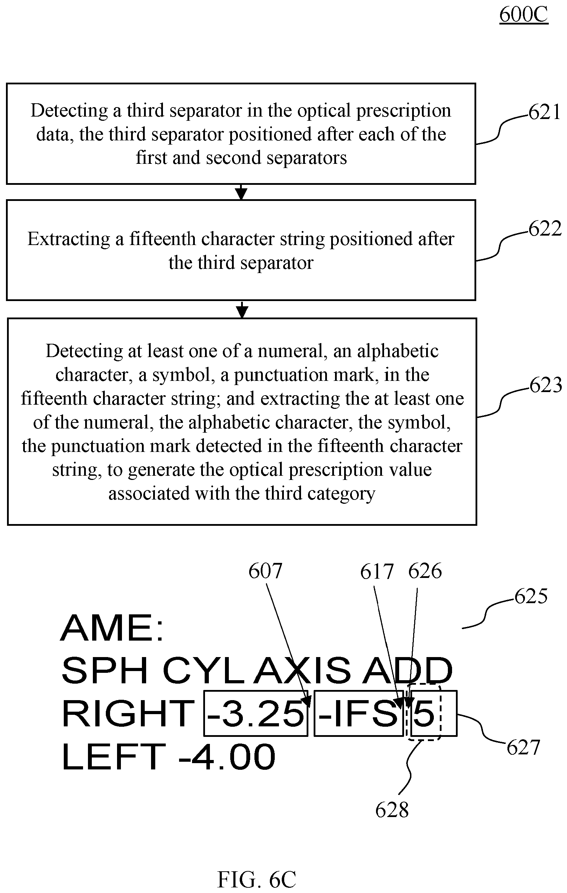

Figure 6C shows a flowchart of method 600C for classifying the portion of the optical prescription data into the third category associated with the subject's cylinder axis data, including an example 625 of the same. Method 600C may include (i.) detecting a third separator in the optical prescription data, the third separator positioned after each of the first and second separators (step 621); and (ii.) extracting a fifteenth character string positioned after the third separator (step 622). As mentioned above, the cylinder axis data is typically positioned immediately after the cylinder data on the optical prescription image. For example, the cylinder axis data may be positioned after the cylinder data, and may be separated by a single separator " " (via a string manipulation function as described above). Accordingly, methods 600A and 600B may be performed prior to method 600C. As shown in example 625, step 621 may include detecting the third separator 626, which is positioned after the first separator 607 and the second separator 617, and extracting the fifteenth character string 627, e.g. "5" positioned after the third separator 626. It is contemplated that step 622 may further include extracting the third separator 626 and/or a keyword associated with the cylinder axis data, if said keyword is present in the optical prescription data.

In some embodiments, method 600C may further include (iii.) detecting at least one of a numeral, an alphabetic character, a symbol and/or a punctuation mark in the fifteenth character string, and extracting the same in the fifteenth character string, to generate the optical prescription value associated with the third category (step 623). As mentioned above, the cylinder axis data which may include 1 to 3 characters may be contained within the fifteenth character string, the extracted one of the numeral, the alphabetic character, the symbol and/or the punctuation mark in the fifteenth character string, may correspond to the optical prescription value for the cylinder axis data of the third category. In an embodiment, the optical prescription value of the cylinder axis data may have a length of less than or equal to 4 characters, preferably 3 characters or less, and may have the optical prescription value 628 of "5", as shown in example 625.

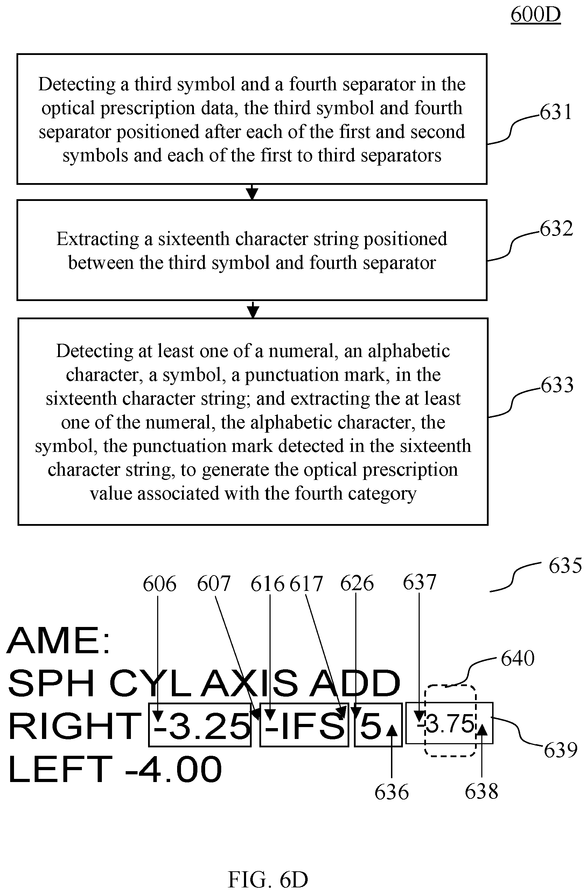

Figure 6D shows a flowchart of method 600D for classifying the portion of the optical prescription data into the fourth category associated with the subject's additional lens power data, including an example 635 of the same. Method 600D may include (i.) detecting a third symbol and a fourth separator in the optical prescription data, the third symbol and fourth separator positioned after each of the first and second symbols and each of the first to third separators (step 631); and (ii.) extracting a sixteenth character string positioned between the third symbol and fourth separator (step 632). The additional lens power data may be a separate measurement, may be typically positioned after the spherical, cylinder and cylinder axis data on the optical prescription image, and may be separated by a single separator 636 e.g. " " (via a string manipulation function as described above). Accordingly, methods 600A to 600C may be performed prior to method 600D. As shown in example 635, step 631 may include detecting the third symbol 637, positioned after each of the first 606 and second 616 symbols, and detecting the fourth separator 638, positioned after each of the first 607, second 617, third 626 separators, and extracting the sixteenth character string 639, e.g. "3.75" positioned between the third symbol 637 and fourth separator 638. It is contemplated that step 632 may further include extracting the third symbol 637 and fourth separator 638.

In some embodiments, method 600D may further include (iii.) detecting at least one of a numeral, an alphabetic character, a symbol and/or a punctuation mark in the sixteenth character string, and extracting the same in the sixteenth character string, to generate the optical prescription value associated with the fourth category (step 633). As mentioned above, the additional lens power data which may include 4 to 6 characters may be contained within the sixteenth string, the extracted one of the numeral, the alphabetic character, the symbol and/or the punctuation mark in the sixteenth character string, may correspond to the optical prescription value for the additional lens power data of the fourth category. In an embodiment, the optical prescription value of the additional lens power data may have a length of less than or equal to 5 characters, and may have the optical prescription value 640 of "3.75" or "-3.75" as shown in example 635.

Referring back to figure 1, method 100 further includes (iv.) determining whether the optical prescription value associated with the respective one of the one or more predetermined categories contains an error, and, if the optical prescription value contains the error, correcting the error within the optical prescription value, to generate a corrected optical prescription value associated with the respective one of the one or more predetermined categories (step 108).

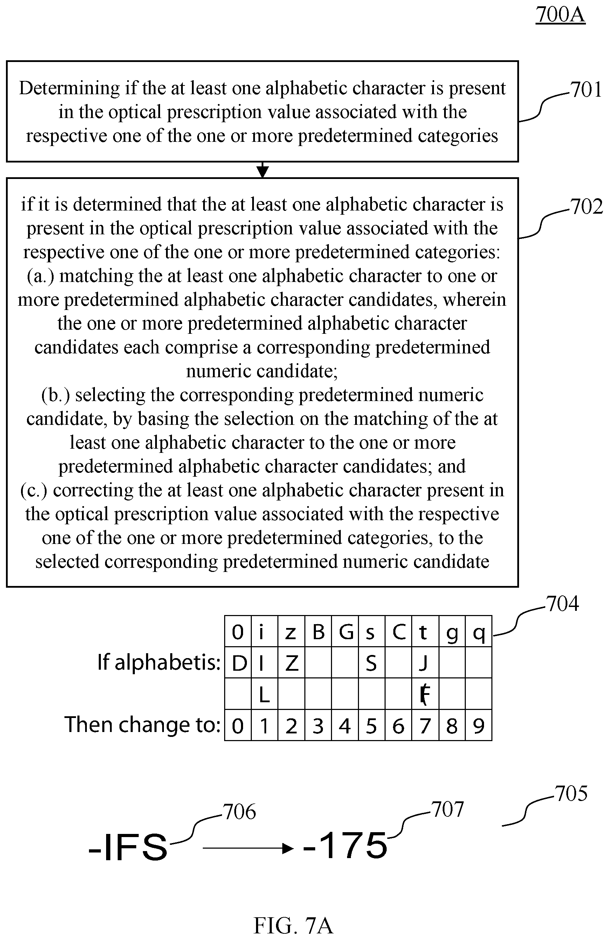

Figures 7A to 7C show exemplary schematic illustrations of methods 700A, 700B, 700C for determining whether the optical prescription value associated with the respective one of the one or more predetermined categories contains an error, and, if the optical prescription value contains the error, correcting the error within the optical prescription value, to generate a corrected optical prescription value associated with the respective one of the one or more predetermined categories, in accordance with the various embodiments of the disclosure.

Figure 7A shows a flowchart of method 700A for determining whether the optical prescription value associated with the respective one of the one or more predetermined categories contains an error, and, if the optical prescription value contains the error, correcting the error within the optical prescription value, to generate a corrected optical prescription value associated with the respective one of the one or more predetermined categories, including an exemplary alphanumeric correction table 704 for correcting the error, and an example 705 of the same. In various embodiments, the optical prescription values associated with the respective one of the one or more predetermined categories may be generated according to any of methods 400A to 600D.

Method 700A may include (i.) determining if the at least one alphabetic character is present in the optical prescription value associated with the respective one of the one or more predetermined categories (step 701); and if it is determined that the at least one alphabetic character is present in the optical prescription value associated with the respective one of the one or more predetermined categories: (a.) matching the at least one alphabetic character to one or more predetermined alphabetic character candidates, wherein the one or more predetermined alphabetic character candidates each comprise a corresponding predetermined numeric candidate; (b.) selecting the corresponding predetermined numeric candidate, by basing the selection on the matching of the at least one alphabetic character to the one or more predetermined alphabetic character candidates; and (c.) correcting the at least one alphabetic character present in the optical prescription value associated with the respective one of the one or more predetermined categories, to the selected corresponding predetermined numeric candidate (step 702).

Referring to alphanumeric correction table 704, and example 705, each alphabetic character that is detected in step 701 may have a corresponding predetermined alphabetic character, which further corresponds to a predetermined numeric candidate. As shown in example 705, step 701 may include detecting the alphabetic characters "I", "F", and "S" in the optical prescription value 706, indicating that one or more errors are present in the optical prescription value 706. As shown in alphanumeric correction table 704, the predetermined alphabetic character "I", "F", "S" may correspond to numeric candidates "1", "7", "5", respectively. Accordingly, step 702 may include (a.) matching the detected alphabetic character "I", "F", "S" to the predetermined alphabetic character "I", "F", "S", in accordance with alphanumeric correction table 704; (b.) selecting the corresponding respective one of the predetermined numeric candidates "1", "7", "5" in accordance with alphanumeric correction table 704; and (c.) correcting the detected alphabetic characters "I", "F", "S" to the selected corresponding predetermined numeric candidate "1", "7", "5", to generate the corrected optical prescription value 707, e.g. "-175".

It is contemplated that method 700A may further include performing the steps 701 and 702 for at least one symbol, e.g. "\"; "|"; "{", "}" that may be present in the optical prescription value associated with the respective one of the one or more predetermined categories. For example, the at least one symbol which may be detected in step 701 may have one or more predetermined symbol candidates, wherein the one or more predetermined symbol candidates each include a corresponding predetermined numeric candidate. Accordingly, step 702 may be performed to correct the at least one symbol present in the optical prescription value to a selected corresponding predetermined numeric candidate, for the respective one of the one or more predetermined categories. In an embodiment, the one or more predetermined symbol candidates and its corresponding predetermined numeric candidates may be stored in the alphanumeric correction table 704 or in a separate table or database.

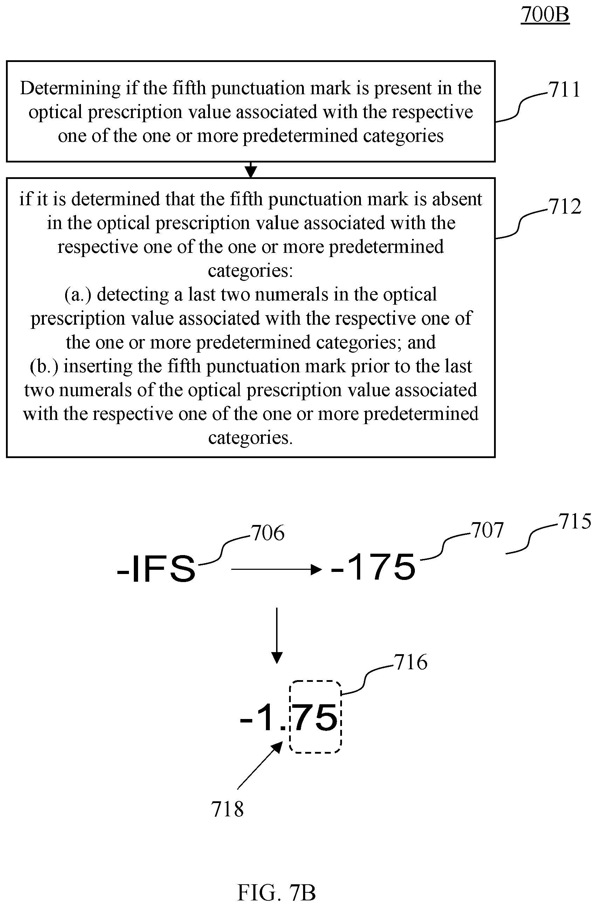

Figure 7B shows a flowchart of method 700B for correcting the error within the optical prescription value, to generate the corrected optical prescription value associated with the respective one of the one or more predetermined categories, including an example 715 of the same. In some embodiments, method 700B may be performed after method 700A for correcting a detected alphabetic character into a selected corresponding predetermined numeric candidate.

Method 700B may include (i.) determining if the fifth punctuation mark is present in the optical prescription value associated with the respective one of the one or more predetermined categories (step 711); and (ii.) if it is determined that the fifth punctuation mark is absent in the optical prescription value associated with the respective one of the one or more predetermined categories: (a.) detecting a last two numerals in the optical prescription value associated with the respective one of the one or more predetermined categories; and (b.) inserting the fifth punctuation mark prior to the last two numerals of the optical prescription value associated with the respective one of the one or more predetermined categories (step 712).

Referring to example 715, the corrected optical prescription value 707 obtained according to method 700A, e.g. "-175" may not include a punctuation mark, e.g. "," or "." (step 711). Accordingly, step 712 may include detecting the last two numerals 716, e.g. "75" in the optical prescription value 707, and inserting a punctuation mark 718, e.g. "," or ".", preferably ".", prior to the last two numerals 716, to generate the further corrected optical prescription value of "-1.75". In some embodiments, method 700B may be applicable to the optical prescription values associated with the first (spherical data), second (cylinder data), fourth (additional lens power data), and the sixth (axial length data) categories.

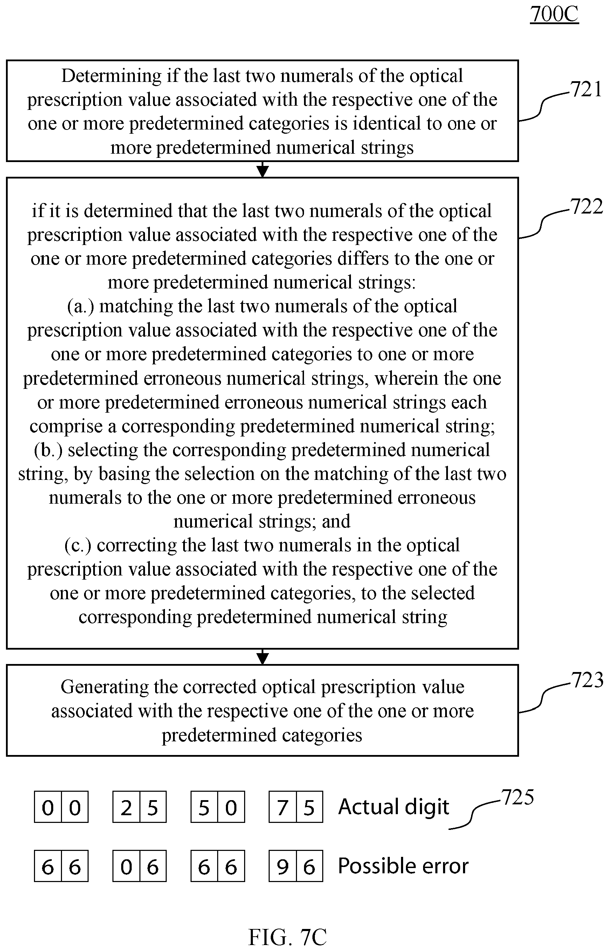

Figure 7C shows a flowchart of method 700C for correcting the error within the optical prescription value, to generate a corrected optical prescription value associated with the respective one of the one or more predetermined categories, including an exemplary numeric correction table 725 for correcting an error in the last two numerals of the optical prescription value. In some embodiments, method 700C may be performed after each of methods 700A and 700B. In an embodiment, method 700C may be the final step for correcting a detected error within the optical prescription value associated with the respective one of the one or more predetermined categories.

Method 700C may include (i.) determining if the last two numerals of the optical prescription value associated with the respective one of the one or more predetermined categories is identical to one or more predetermined numerical strings (step 721); (ii.) if it is determined that the last two numerals of the optical prescription value associated with the respective one of the one or more predetermined categories differs to the one or more predetermined numerical strings: (a.) matching the last two numerals of the optical prescription value associated with the respective one of the one or more predetermined categories to one or more predetermined erroneous numerical strings, wherein the one or more predetermined erroneous numerical strings each comprise a corresponding predetermined numerical string; (b.) selecting the corresponding predetermined numerical string, by basing the selection on the matching of the last two numerals to the one or more predetermined erroneous numerical strings; and (c.) correcting the last two numerals in the optical prescription value associated with the respective one of the one or more predetermined categories, to the selected corresponding predetermined numerical string (step 722); and (iii.) generating the corrected optical prescription value associated with the respective one of the one or more predetermined categories (step 723).

Referring to numeric correction table 725, the last two numerals of the optical prescription value associated with the respective one of the one or more predetermined categories, which may be obtained in accordance with method 700B and determined in step 721 may have a corresponding predetermined erroneous numerical string, which further corresponds to a predetermined numerical string. For example, step 721 may include detecting the last two numerals "66" in the optical prescription value, indicating that one or more errors are present in the optical prescription value. As shown in numeric correction table 725, the predetermined erroneous numerical string "66" may correspond to the predetermined numerical string "00". Accordingly, step 722 may include (a.) matching the detected erroneous numerical string "66" to the predetermined numerical string "00", in accordance with numeric correction table 725; (b.) selecting the corresponding respective one of the predetermined numerical string "00", in accordance with numeric correction table 725, and (c.) correcting the detected last two numerals "66" to the selected corresponding predetermined numerical string "00". Step 723 may thus include generating the corrected optical prescription value where errors in the last two numerals may be corrected to its corresponding predetermined numerical string. In an embodiment, method 700C may be applicable to the optical prescription values associated with the first (spherical data), second (cylinder data), fourth (additional lens power data), and the sixth (axial length data) categories.

While exemplary numeric correction table 725 shows 4 possible predetermined numerical strings in 0.25 D increments, the numeric correction table in accordance with various embodiments of the disclosure may include numerical strings in any increment. In some embodiments, the predetermined numerical strings may be in 0.12 D increments, 0.05 D increments, or 0.01 D increments.

In various embodiments, the methods of the present disclosure may further include emitting an alert, the various embodiments of which is described with reference to figures 8A and 8B.

Figure 8A shows a flowchart of method 800A for determining if the corrected prescription value associated with the one or more predetermined categories falls within a reference range of values associated with the respective one of the one or more predetermined categories. Method 800A includes (i.) determining if the corrected prescription value associated with the one or more predetermined categories falls within a reference range of values associated with the respective one of the one or more predetermined categories (step 801). In some embodiments, step 801 may include determining if the corrected optical prescription value associated with the respective one of the one or more predetermined categories, which may be generated in accordance with any of methods 700A to 700C, falls within a reference range of values. In an embodiment, the reference range of values may be associated with myopia in children or adolescents, and may include a predetermined range associated with the respective category. In some non-limiting embodiments, the first category associated with the spherical data may have a reference range of values ranging from -0.00 to -10.00 D; the second category associated with the cylinder data may have a reference range of values ranging from 0.00 to ±3.50 D; the third category associated with the cylinder axis data may have a reference range of values ranging from 0 to 180 °; the fourth category associated with the additional lens power data may have a reference range of values ranging from ±0.50 to ±0.35 D; the fifth category associated with the pupil distance data may have a reference range of values, ranging from 30 to 80 mm, preferably 50 to 72 mm for binocular measurements, and ranging from 15 to 40 mm, preferably 25 to 36 mm for monocular measurements; and the sixth category associated with the axial length data may have a reference range of values ranging from 20.00 to 27.00 mm. The reference range of values have been provided as exemplary reference range of values associated with the respective one of the first to sixth category, which may be dependent on the subject's age and gender, as would be known to those skilled in the art.

Method 800A may further include (ii.) emitting a first alert, if it is determined that the corrected optical prescription value falls outside the reference range of values associated with the respective one of the one or more predetermined categories (step 802). For example, the first alert may notify the user or the eye care practitioner that an incorrect optical prescription may have been provided, or that the corrected optical prescription value may include an error that was not corrected in accordance with methods 700A to 700C of the disclosure. In other words, the first alert may suggest to the user that the corrected optical prescription value falls outside the predetermined reference range of values, and may include an error.

Figure 8B shows a flowchart of method 800B for determining if a difference between the corrected optical prescription value associated with the respective one of the one or more predetermined categories and a respective previous optical prescription value associated with the respective one of the one or more predetermined categories is greater than a predefined threshold value. Method 800B may include (i.) calculating a difference between the corrected optical prescription value associated with the respective one of the one or more predetermined categories with a respective previous optical prescription value associated with the respective one of the one or more predetermined categories (step 811). For example, step 811 may include comparing the corrected optical prescription value associated with the respective one of the one or more predetermined categories, which may be generated in accordance with any of methods 700A to 700C, with the subject's previous optical prescription value associated with the respective one of the one or more predetermined categories. In an embodiment, at least one of the subject's previous optical prescription values associated with the one or more predetermined categories may be stored in a memory, or a database on an external server, for comparison purposes.

Method 800B may further include (ii.) emitting a second alert, if it is determined that the difference is greater than a predefined threshold value associated with the respective one of the one or more predetermined categories (step 812). For example, the calculated difference obtained in step 812 may be compared against a predefined threshold value associated with any of the first to sixth categories, e.g. ±5 D for the first, second, fourth categories associated with the spherical, cylinder and additional lens power data, respectively; ±10 ° for the third category associated with the cylinder axis data; and ±10 mm for the fifth and sixth categories associated with the pupil distance and axial length data, respectively. For example, the second alert may notify the user or the eye care practitioner that a discrepancy between the corrected optical prescription value and a previous measurement is too great, suggesting that an incorrect optical prescription may have been provided, or that the corrected optical prescription value may include an error that was not corrected in accordance with methods 700A to 700C of the disclosure.

According to various embodiments, correcting the error within the optical prescription value associated with the respective one of the one or more predetermined categories to generate the corrected optical prescription value (step 108) of method 100, may be based on a machine learning algorithm. For example, one or more neural networks may be trained by inputting a series of optical prescription values associated with the respective one of the one or more predetermined categories, identifying possible errors in the numerals, alphabetic characters, and/or the last two numerals of said optical prescription value, and its corresponding predetermined numeric candidate or numerical string, to build a correlation table, e.g. alphanumeric correction table 704 or numeric correction table 725, or any database containing information on the relationship between the possible errors and the corresponding predetermined numeric candidate or numerical string. In an embodiment, said correlation table or database be based on studying a set of optical prescription images and establishing the correlation between the possible errors in the numerals, alphabetic characters, and/or the last two numerals of said optical prescription value, and its corresponding predetermined numeric candidate or numerical string.

According to various embodiments, the machine learning algorithm may be selected from the group consisting of supervised, semi-supervised and unsupervised learning, for example: a support vector machine (SVM), a Naive Bayes classification, a random forest, an artificial neural network, a decision tree, a K-means, learning vector quantization, self-organizing maps, graphical models, preferably, regression algorithms, e.g. linear, multi-variate, logistic, association rule learning, deep learning, dimensionality reduction and ensemble selection algorithms. In an embodiment, the machine learning algorithm may be a supervised learning algorithm, for example, a convolutional neural network.

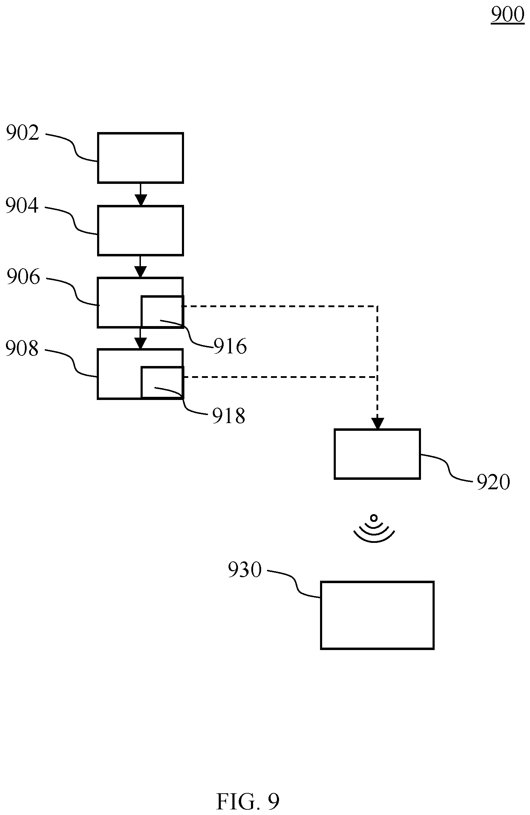

According to another aspect of the disclosure, there is provided a system for reading an optical prescription on an optical prescription image. Figure 9 shows a schematic illustration of a system 900 for reading an optical prescription on an optical prescription image, by way of example. System 900 may be based on and configured to execute the steps of method 100, and repeated description will be omitted for brevity.

System 900 includes (i.) means for detecting a region comprising the optical prescription on the optical prescription image 902, and (ii.) means for extracting the optical prescription and converting the optical prescription into machine-encoded optical prescription data 904. Each of the said means 902, 904 may include at least one circuit, e.g. microprocessor, for example, an image sensor for detecting said region on the optical prescription image. The at least one circuit may be configured, e.g. include instructions, to detect the at least one keyword, and to extract the optical prescription and to convert the optical prescription into machine-encoded optical prescription data. In an embodiment, the means for extracting the optical prescription and converting the optical prescription into machine-encoded optical prescription data 904 may be based on OCR technologies.

System 900 further includes (iii.) means for classifying a portion of the optical prescription data into one or more predetermined categories, to generate an optical prescription value associated with a respective one of the one or more predetermined categories 906; and (iv.) means for determining whether the optical prescription value associated with the respective one of the one or more predetermined categories contains an error, and, if the optical prescription value contains the error, correcting the error within the optical prescription value associated with the respective one of the one or more predetermined categories, to generate a corrected optical prescription value associated with the respective one of the one or more predetermined categories 908. Each of the said means 906, 908 may include at least one circuit, e.g. microprocessor, which may be configured to store and execute instructions, to perform the steps of any of methods 400A to 600D as described with reference to figures 4A to 6D for classifying the portion of the optical prescription data into one or more predetermined categories, and to perform the steps of any of methods 700A to 700C as described with reference to figures 7A to 7C for determining whether the optical prescription value associated with the respective one of the one or more predetermined categories contains an error, and, if the optical prescription value contains the error, correcting the error within the optical prescription value associated with the respective one of the one or more predetermined categories, to generate a corrected optical prescription value associated with the respective one of the one or more predetermined categories. In various embodiments, system 900 may be further configured to perform the steps of methods 800A and 800B, as described with reference to figures 8A and 8B for emitting a first and/or second alert.

In system 900, the circuit associated with the means for classifying the portion of the optical prescription data into one or more predetermined categories, to generate the optical prescription value associated with the respective one of the one or more predetermined categories 906 may further include a memory 916 configured to store the optical prescription value associated with the respective one of the one or more predetermined categories. In addition, the circuit associated with the means for determining whether the optical prescription value associated with the respective one of the one or more predetermined categories contains the error, and correcting the error within the optical prescription value 908, may further include a memory 918 configured to store the corrected optical prescription value associated with the respective one of the one or more predetermined categories.

Alternatively, or in addition, system 900 may further include a memory 920 for storing the classified optical prescription value associated with the respective one of the one or more predetermined categories, and the corrected optical prescription value associated with the respective one of the one or more predetermined categories.

In various embodiments, the circuit associated with the means for classifying the portion of the optical prescription data into one or more predetermined categories to generate the optical prescription value associated with the respective one of the one or more predetermined categories 906, and the circuit associated with the means for determining whether the optical prescription value associated with the respective one of the one or more predetermined categories contains the error, and correcting the error within the optical prescription value 908, may be further configured to send the classified optical prescription value associated with the respective one of the one or more predetermined categories stored in memory 916, and the corrected optical prescription value associated with the respective one of the one or more predetermined categories stored in memory 918, to an external server 930. Alternatively, system 900 may be configured to send the classified optical prescription value associated with the respective one of the one or more predetermined categories, and the corrected optical prescription value associated with the respective one of the one or more predetermined categories, which may be stored in memory 920, to the external server 930.

According to various embodiments, the classified optical prescription value associated with the respective one of the one or more predetermined categories, and the corrected optical prescription value associated with the respective one of the one or more predetermined categories, may be sent to the external server 930 at predetermined time intervals, or may be sent upon request by the external server 930, and may be transmitted according to a pre-defined wireless communication protocol. Examples of the pre-defined wireless communication protocols include: global system for mobile communication (GSM), enhanced data GSM environment (EDGE), wideband code division multiple access (WCDMA), code division multiple access (CDMA), time division multiple access (TDMA), wireless fidelity (Wi-Fi), voice over Internet protocol (VoIP), worldwide interoperability for microwave access (Wi-MAX), Wi-Fi direct (WFD), an ultra-wideband (UWB), infrared data association (IrDA), Bluetooth, ZigBee, SigFox, LPWan, LoRaWan, GPRS, 3G, 4G, LTE, and 5G communication systems. Accordingly, each of the circuits 906, 908, 920 may include the necessary hardware required to support the wireless transmission. Alternatively, it is contemplated that said optical prescription values may be transmitted via wired means.

According to various embodiments, a computer program product may include instructions to cause the system 900 to execute the steps of methods 100, and 400A to 800B. The steps of methods 100, and 400A to 800B may be used for reading the optical prescription on the optical prescription image, in particular, to classify a portion of the optical prescription data into one or more predetermined categories, to generate an optical prescription value associated with a respective one of the one or more predetermined categories; and to determining whether the optical prescription value associated with the respective one of the one or more predetermined categories contains an error, and, if the optical prescription value contains the error, correcting the error within the optical prescription value associated with the respective one of the one or more predetermined categories, to generate a corrected optical prescription value associated with the respective one of the one or more predetermined categories.

Accordingly, the methods and system of the present disclosure provides means to accurately and efficiently read an optical prescription on an optical prescription image, to aid the user, e.g. subject or subject's parents, in the understanding and/or monitoring of their visual health, in particular, for the myopia onset or progression.

### EXAMPLES

The system 900 and methods 100, 400A to 800B, herein disclosed are further illustrated in the following examples, which are provided by way of illustration and are not intended to be limiting the scope of the present disclosure.

### Example 1. Handwritten optical prescription including Chinese characters

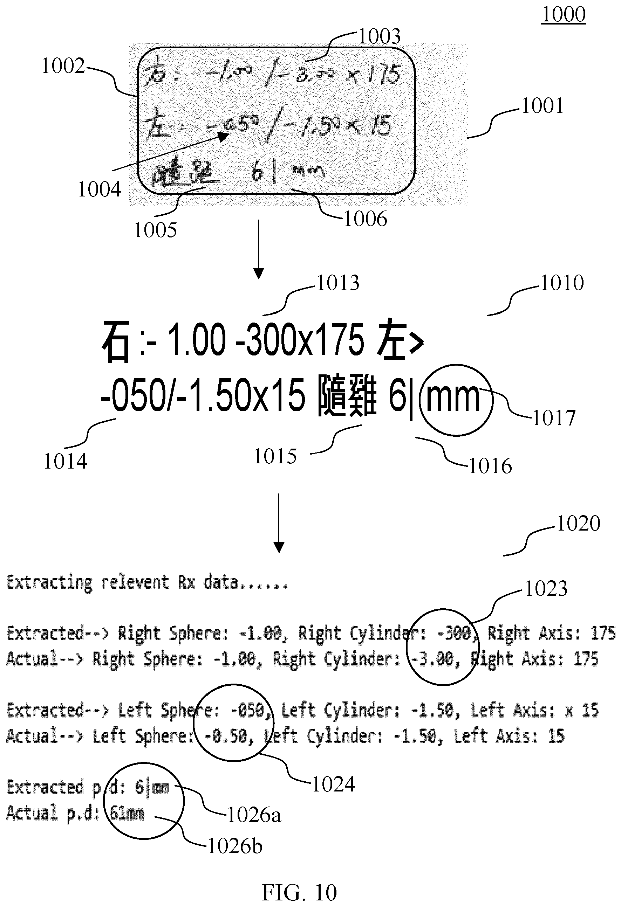

Figure 10 shows an example 1000 for reading a handwritten optical prescription on an optical prescription image 1001. In some embodiments, the optical prescription on the optical prescription image 1001 may include Chinese characters, for example, the at least one keyword may be written in Chinese.

In accordance with various embodiments of the disclosure, the machine-encoded optical prescription data 1010 may be obtained by (i.) detecting the region 1002 comprising the optical prescription on the optical prescription image 1001 (step 102), by detecting at least one keyword, e.g. " 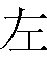 "; " 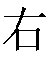 "; " 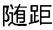 " and the portion of the optical prescription associated with the at least one keyword; and (ii.) extracting the at least one keyword and the portion of the optical prescription associated with the at least one keyword within region 1002, and converting said at least one keyword and said portion of the optical prescription into machine-encoded optical prescription data (step 104). In some embodiments, step 104 may be performed in accordance with OCR techniques.

As shown in figure 10, the machine-encoded optical prescription data 1010 may be incorrectly converted. For example, the punctuation mark, e.g. "." in the optical prescription associated with the subject's right eye cylinder data 1003, was omitted in the optical prescription data associated with the cylinder data 1013, e.g. "-300". Similarly, the punctuation mark, e.g. "." in the optical prescription associated with the subject's left eye spherical data 1004 was omitted in the optical prescription data associated with the spherical data 1014, e.g. "-050". In another example, the keyword associated with the pupil distance data 1005, e.g. " 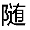 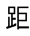 " and the portion of the optical prescription 1006, e.g. "61mm" associated with said keyword 1005 was incorrectly converted into the erroneous keyword associated with the pupil distance data 1015, e.g. " 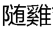 " and the optical prescription data associated with the pupil distance data 1016, e.g. "6*l*mm" ("1" indicating lowercase alphabetic character L).

Method 100 further includes (iii.) classifying a portion of the optical prescription data 1010 into one or more predetermined categories, to generate an optical prescription value associated with a respective one of the one or more predetermined categories (step 106), which may be performed in accordance with any of methods 400A to 600D as described with reference to figures 4A to 6D, and repeated description will be omitted for brevity. The classified optical prescription values may be seen in the rows titled "Extracted" in the set of optical prescription values 1020.

For example, the optical prescription data associated with the fifth category for the pupil distance data may be classified in accordance with method 500E. In an embodiment, the keyword associated with the fifth category for the pupil distance data 1005, e.g. " 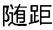 " may include a corresponding one or more erroneous keywords associated with said keyword 1005, e.g. " 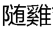 ". As such, the erroneous keyword associated with the fifth category for the pupil distance data 1015, e.g. " 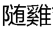 " may be detected, and an eighteenth character string comprising 5 characters, e.g. " 6*l*mm", positioned after the erroneous keyword associated with the fifth category for the pupil distance data 1015 may be extracted. The optical prescription value associated with the fifth category for the pupil distance data 1026a may be generated by detecting at least one numeral, alphabetic character, symbol and/or punctuation mark in the eighteenth character string, e.g. "6*l*" having a length less than or equal to 2 characters. In another embodiment, the erroneous keyword associated with the fifth category for the pupil distance data 1015, e.g. " 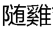 " may not be detected. Instead, method 500E may include the detection of a unit associated with the fifth category 1017, e.g. "mm" and extracting the nineteenth character string positioned before the unit associated with the fifth category 1017, e.g. "6*l*" having a length less than or equal to 2 characters, as the optical prescription value associated with the fifth category for the pupil distance data 1026a.

Method 100 further includes determining whether the optical prescription value associated with the respective one of the one or more predetermined categories contains an error, and, if the optical prescription value contains the error, correcting the error within the optical prescription value, to generate a corrected optical prescription value associated with the respective one of the one or more predetermined categories (step 108), in accordance with methods 700A to 700C of the disclosure. The corrected optical prescription values may be seen in the rows titled "Actual" in the set of optical prescription values 1020.

For example, method 700B may be performed to insert a punctuation mark, e.g. "." prior to the last two numerals of the optical prescription value associated with the second category for the cylinder data, to generate the corrected optical prescription value 1023, e.g. "-3.00"; and to insert a punctuation mark, e.g. "." prior to the last two numerals of the optical prescription value associated with the first category for the spherical data, to generate the corrected optical prescription value 1024, e.g. "-0.50". In another example, method 700A may be performed to correct the alphabetic character "l" in the optical prescription value associated with the fifth category for the pupil distance data 1026a, e.g. "6*l*", into its corresponding predetermined numeric candidate, e.g. "1", in accordance with exemplary alphanumeric correction table 704, to generate the corrected optical prescription value associated with the fifth category for the pupil distance data 1026b, e.g. "61".

### Example 2. Handwritten optical prescription including additional lens power measurements

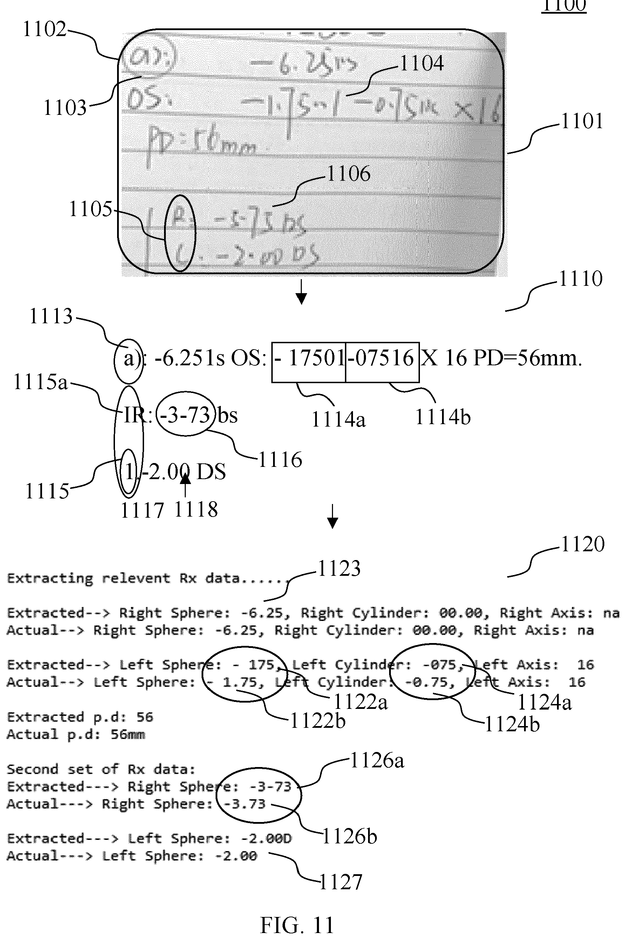

Figure 11 shows another example 1100 for reading a handwritten optical prescription on an optical prescription image 1101.

In accordance with various embodiments of the disclosure, the machine-encoded optical prescription data 1110 may be obtained by (i.) detecting the region 1102 comprising the optical prescription on the optical prescription image 1101 (step 102), by detecting at least one keyword, e.g. "OD"; "OS"; "PD"; "R"; "L", and the portion of the optical prescription associated with the at least one keyword; and (ii.) extracting the at least one keyword and the portion of the optical prescription associated with the at least one keyword within region 1102, and converting said at least one keyword and said portion of the optical prescription into optical prescription data (step 104). In some embodiments, step 104 may be performed in accordance with OCR techniques.

Referring to figure 11, the optical prescription data 1110 may include one or more errors. For example, the keyword associated with the subject's right eye spherical data 1103, e.g. "OD" was incorrectly converted into the erroneous keyword associated with the subject's right eye spherical data 1113, e.g. "a)"; the portion of the optical prescription data 1104 associated with the keyword for the subject's left eye spherical data, e.g. "OS" was incorrectly converted into the erroneous optical prescription data 1114a, 1114b, e.g. "-17501-075146"; the keywords associated with the subject's right and left eye additional lens power data 1105, e.g. "R"; "L" was incorrectly converted into the erroneous keyword associated with the subject's right and left eye additional lens power data 1115, e.g. "IR"; "1"; and the portion of the optical prescription data 1106 associated with the subject's right eye additional lens power data, e.g. "-3.75" was incorrectly converted into the erroneous optical prescription data 1116, e.g. "-3-75".

Method 100 further includes (iii.) classifying a portion of the machine-encoded optical prescription data 1101 into one or more predetermined categories, to generate an optical prescription value associated with a respective one of the one or more predetermined categories (step 106), which may be performed in accordance with any of methods 400A to 600D as described with reference to figures 4A to 6D, and repeated description will be omitted for brevity. The classified data may be seen in the rows titled "Extracted" in the set of optical prescription values 1120.

For example, the optical prescription data associated with the first category for the right eye spherical data may be classified in accordance with method 500A. For example, the keyword associated with the first category for the right eye spherical data 1103, e.g. "OD" may include a corresponding one or more erroneous keywords associated with said keyword 1103, e.g. "a)". As such, the erroneous keyword associated with the first category for right eye spherical data 1113, e.g. "a)" may be detected, and a ninth character string comprising 7 characters, e.g. " -6.251", positioned after the erroneous keyword associated with the first category for the right eye spherical data 1113 may be extracted. The optical prescription value associated with the first category for the right eye spherical data 1123 may be generated by detecting at least one numeral, alphabetic character, symbol and/or punctuation mark in the ninth character string, e.g. "-6.25" having a length less than or equal to 5 characters.

Similarly, the optical prescription data associated with the fourth category for the right eye additional lens power data may be classified in accordance with method 500D. The keyword associated with the fourth category for the right eye additional lens power data 1105, e.g. "R" may include a corresponding one or more erroneous keywords associated with said keyword 1105, e.g. "IR". As such, the erroneous keywords associated with the fourth category for right eye additional lens power data 1115a, e.g. "IR" may be detected, and an eleventh character string comprising 7 characters, e.g. " -3-73 ", positioned after the erroneous keyword associated with the fourth category for the right eye additional lens power data 1115a may be extracted. The optical prescription value associated with the fourth category for the right eye additional lens power data 1126a may be generated by detecting at least one numeral, alphabetic character, symbol and/or punctuation mark in the eleventh character string, e.g. "-3-73" having a length less than or equal to 5 characters

In an embodiment of example 1100, the optical prescription data associated with the fourth category for the left eye additional lens power data may be classified in accordance with method 600D. For example, the optical prescription data associated with the fourth category for the left eye additional lens power data may include detecting the third symbol 1117 and a fourth separator 1118 in the optical prescription data 1110 and extracting the sixteenth character string, e.g. "-2.00" positioned between the third symbol 1116 and fourth separator 1118. Method 600D may further include extracting the third symbol 1116 and/or the fourth separator. The optical prescription value associated with the fourth category for the left eye additional lens power data 1127 may be generated by detecting at least one numeral, alphabetic character, symbol and/or punctuation mark in the sixteenth character string, e.g. "-2.00" having a length less than or equal to 5 characters. Further, method 600D may include adding the unit associated with the fourth category, e.g. "D (dioptre)" in the optical prescription value associated with the fourth category for the left eye additional lens power data 1127.

In another embodiment of example 1100, the optical prescription data associated with the first category for the left eye spherical data, and the second category for the cylinder data, may be classified in accordance with methods 500A and 500B. For example, method 500A may include detecting the keyword associated with the first category for the left eye spherical data, e.g. "OS", and extracting the ninth character string having the second predefined length of 7 characters, e.g. "- 17501", and method 500B may include detecting the tenth character string having the second predefined length of 7 characters, e.g. "-07516 " positioned after the portion of the optical prescription data classified into the first category, e.g. ninth character string. The optical prescription values associated with the first category for the left eye spherical data 1122a and the second category for the cylinder data 1124a may be generated by detecting at least one numeral, alphabetic character, symbol and/or punctuation mark in the ninth and tenth character strings, respectively, e.g. "- 175" and " 075", having a length less than or equal to 5 characters.

Method 100 further includes (iv.) determining whether the optical prescription value associated with the respective one of the one or more predetermined categories contains an error, and, if the optical prescription value contains the error, correcting the error within the optical prescription value, to generate a corrected optical prescription value associated with the respective one of the one or more predetermined categories (step 108), which may be performed in accordance with any of methods 700A to 700C. The corrected optical prescription values may be seen in the rows titled "Actual" in the set of optical prescription values 1120.

For example, method 700B may be performed to insert a punctuation mark, e.g. "." in the optical prescription values associated with first category for the left spherical data 1122a to generate the corrected optical prescription value associated with the first category for the left spherical data 1122b, e.g. "-1.75"; and in the optical prescription values associated with second category for the left spherical data cylinder data 1124a to generate the corrected optical prescription value associated with the second category for the cylinder data 1124b, e.g. "-0.75".

In another example, method 700A may be performed to replace the symbol, e.g. "-" in the optical prescription value associated with the fourth category for the right eye additional lens power data 1126a with a punctuation mark, e.g. "." to generate the corrected optical prescription value associated with the fourth category for the right eye additional lens power data 1126b. For example, said symbol may correspond to a corresponding predetermined punctuation, the correlation of which may be stored in exemplary alphanumeric correction table 704, or in a separate table or database on an external server.

### Example 3. Handwritten optical prescription on a printed prescription form

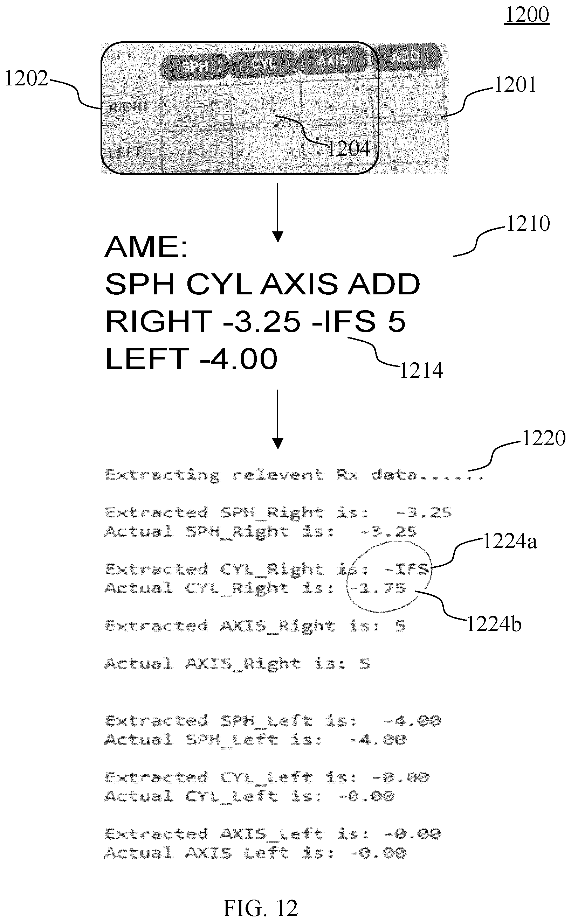

Figure 12 shows another example 1200 for reading a handwritten optical prescription on an optical prescription image 1201. In this example 1200, the handwritten optical prescription may be written on a printed prescription form.

In accordance with various embodiments of the disclosure, the machine-encoded optical prescription data 1210 may be obtained by (i.) detecting the region 1202 comprising the optical prescription on the optical prescription image 1201 (step 102), by detecting at least one keyword, e.g. "RIGHT"; "LEFT"; "SPH"; "CYL"; "AXIS", and the portion of the optical prescription associated with the at least one keyword; and (ii.) extracting the at least one keyword and the portion of the optical prescription associated with the at least one keyword within region 1202, and converting said at least one keyword and said portion of the optical prescription into optical prescription data (step 104). In some embodiments, step 104 may be performed in accordance with OCR techniques.

Referring to figure 12, the optical prescription data 1210 may include one or more errors. For example, the optical prescription data for the cylinder data 1204, e.g. "-175" was incorrectly converted into erroneous optical prescription data 1214, e.g. "-IFS" in the optical prescription data 1210.

Method 100 further includes (iii.) classifying a portion of the machine-encoded optical prescription data 1210 into one or more predetermined categories, to generate an optical prescription value associated with a respective one of the one or more predetermined categories (step 106), which may be performed in accordance with any of methods 400A to 600D as described above, and repeated description will be omitted for brevity. The classified data may be seen in the rows titled "Extracted" in the set of optical prescription values 1220.

For example, the optical prescription value associated the second category for cylinder data 1224a may be generated by detecting at least one numeral, alphabetic character, symbol and/or punctuation mark in an extracted character string, e.g. " -IFS" and may have a length less than or equal to 5 characters. The optical prescription associated the second category for cylinder data 1224a may be generated in accordance with any of methods 400B, 500B, 600B, preferably method 600B.

Method 100 further includes (iv.) determining whether the optical prescription value associated with the respective one of the one or more predetermined categories contains an error, and, if the optical prescription value contains the error, correcting the error within the optical prescription value, to generate a corrected optical prescription value associated with the respective one of the one or more predetermined categories (step 108), which may be performed in accordance with any of methods 700A to 700C as described above. The corrected optical prescription values may be seen in the rows titled "Actual" in the set of optical prescription values 1220.

For example, method 700A may be performed to correct the detected alphabetic characters "I", "F", "S" in the optical prescription value associated with the second category for the cylinder data 1124a, by selecting corresponding predetermined numeric candidates in accordance with exemplary alphanumeric correction table 704, e.g. "I" to "1"; "F" to "7"; "S" to "5" to obtain "-175". In addition, method 700B may be performed for inserting a punctuation mark, e.g. "." prior to the last two numerals to generate the corrected optical prescription value associated with the second category for the cylinder data 1224b, e.g. "-1.75".

Accordingly, the methods and system of the present disclosure provides for reading an optical prescription on an optical prescription image, specifically by classifying the optical prescription into various categories, and correcting any errors in the optical prescription, thereby providing an efficient and accurate approach for reading optical prescriptions on optical prescription images.

The scope of the disclosure is thus indicated by the appended claims and all changes which come within the meaning and range of equivalency of the claims are therefore intended to be embraced.

## Claims

1. A computer-implemented method (100) for reading an optical prescription on an optical prescription image comprising
(i.) detecting a region comprising the optical prescription on the optical prescription image (102);
(ii.) extracting the optical prescription and converting the optical prescription into machine-encoded optical prescription data (104);
(iii.) classifying a portion of the optical prescription data into one or more predetermined categories, to generate an optical prescription value associated with a respective one of the one or more predetermined categories (106); and
(iv.) determining whether the optical prescription value associated with the respective one of the one or more predetermined categories contains an error being an incorrect conversion of an alphabetic character, and, if the optical prescription value contains the error, correcting the error within the optical prescription value by selecting a corresponding predetermined numeric candidate, to generate a corrected optical prescription value associated with the respective one of the one or more predetermined categories (108), wherein
(i.) detecting the region comprising the optical prescription on the optical prescription image (102) comprises detecting at least one keyword and the portion of the optical prescription data associated with the at least one keyword, wherein detecting the at least one keyword comprises detecting the at least one keyword and a corresponding one or more erroneous keywords associated with a respective one of the at least one keyword; and
(ii.) extracting the optical prescription (102) comprises extracting the at least one keyword and the portion of the optical prescription data associated with the at least one keyword.

2. The method (100) of claim 1, wherein the one or more predetermined categories comprises
(i.) a first category associated with a subject's spherical data;
(ii.) a second category associated with the subject's cylinder data;
(iii.) a third category associated with the subject's cylinder axis data;
(iv.) a fourth category associated with the subject's additional lens power data;
(v.) a fifth category associated with the subject's pupil distance data; and
(vi.) a sixth category associated with the subject's axial length data.

3. The method (400A) of claim 2, wherein classifying the portion of the optical prescription data into the first category associated with the subject's spherical data comprises
(i.) detecting a first punctuation mark in the optical prescription data (401); and
(ii.) extracting a first character string positioned before the first punctuation mark, and a second character string positioned after the first punctuation mark, wherein the first and second character strings each have a first predefined length (402);
(iii.) detecting a first keyword from among the at least one keyword, the first keyword associated with the first category (501); and
(iv.) extracting a ninth character string positioned after the first keyword, wherein the ninth character string has a second predefined length (502);
(v.) detecting a first symbol and a first separator in the optical prescription data (601); and
(vi.) extracting a thirteenth character string positioned between the first symbol and the first separator (602);
or any combination of the above, and
wherein the first predefined length comprises 3 characters, and the second predefined length comprises 7 characters.

4. The method (400B) of claim 3, wherein classifying the portion of the optical prescription data into the second category associated with the subject's cylinder data comprises
(i.) detecting a second punctuation mark in the optical prescription data, the second punctuation mark positioned after the first punctuation mark (411); and
(ii.) extracting a third character string positioned before the second punctuation mark, and a fourth character string positioned after the second punctuation mark, wherein the third and fourth character strings each have the first predefined length (412);
(iii.) detecting a tenth character string positioned after the portion of the optical prescription data classified into the first category (511); and
(iv.) extracting the tenth character string, wherein the tenth character string has the second predefined length (512);
(v.) detecting a second symbol and a second separator in the optical prescription data, the second symbol and second separator positioned after the first symbol and the first separator (611); and
(vi.) extracting a fourteenth character string positioned between the second symbol and the second separator (612);
or any combination of the above.

5. The method (400C) of claim 4, wherein classifying the portion of the optical prescription into the fourth category associated with the subject's additional lens power data comprises
(i.) detecting a third punctuation mark in the optical prescription data, the third punctuation mark positioned after each of the first and second punctuation marks (421); and
(ii.) extracting a fifth character string positioned before the third punctuation mark, and a sixth character string positioned after the third punctuation mark, wherein the fifth and sixth character strings each have the first predefined length (422);
(iii.) detecting a second keyword from among the at least one keyword, the second keyword associated with the fourth category (531); and
(iv.) extracting an eleventh character string positioned after the second keyword, wherein the eleventh character string has the second predefined length (532);
or any combination of the above.

6. The method (400D) of claim 5, wherein classifying the portion of the optical prescription data into the sixth category associated with the subject's axial length data comprises
(i.) detecting a fourth punctuation mark in the optical prescription data, the fourth punctuation mark positioned after each of the first, second and third punctuation marks (431); and
(ii.) extracting a seventh character string positioned before the fourth punctuation mark, and an eighth character string positioned after the fourth punctuation mark, wherein the seventh and eighth character strings each comprise 2 characters (432);
(iii.) detecting a third keyword from among the at least one keyword, the third keyword associated with the sixth category (551);
(iv.) extracting a twelfth character string positioned after the third keyword, wherein the twelfth character string has the second predefined length (552); and further comprising
(v.) determining if a unit associated with the sixth category is present in the twelfth character string;
(vi.) if it is determined that the unit associated with the sixth category is present in the twelfth character string: extracting a twentieth character string positioned before the unit associated with the sixth category, to generate the optical prescription value associated with the sixth category (554);
or any combination of the above.

7. The method (600C) of any one of claims 4 to 6, wherein classifying the portion of the optical prescription data into the third category associated with the subject's cylinder axis data comprises
(i.) detecting a third separator in the optical prescription data, the third separator positioned after each of the first and second separators (621); and
(ii.) extracting a fifteenth character string positioned after the third separator (622);
(iii.) detecting a seventeenth character string positioned after the portion of the optical prescription data classified into the second category (521); and
(iv.) extracting the seventeenth character string, wherein the seventeenth character string comprises 6 characters (522);
or any combination of the above.

8. The method (600D) of claim 7, wherein classifying the portion of the optical prescription into the fourth category associated with the subject's additional lens power data comprises
(i.) detecting a third symbol and a fourth separator in the optical prescription data, the third symbol and the fourth separator positioned after each of the first and second symbols and each of the first to third separators (631);
(ii.) extracting a sixteenth character string positioned between the third symbol and the fourth separator (632).

9. The method (500E) of any one of claims 2 to 8, wherein classifying the portion of the optical prescription into the fifth category associated with the subject's pupil distance data comprises
(i.) detecting a fourth keyword from among the at least one keyword, the fourth keyword associated with the fifth category (541);
(ii.) extracting an eighteenth character string positioned after the fourth keyword,
wherein the eighteenth character string comprises 5 characters (542);
(iii.) determining if a unit associated with the fifth category is present in the eighteenth character string;
(iv.) if it is determined that the unit associated with the fifth category is present in the eighteenth character string:
extracting a nineteenth character string positioned before the unit associated with the fifth category, to generate the optical prescription value associated with the fifth category (543).

10. The method (400A, 400B, 400C, 400D, 500A, 500B, 500C, 500D, 500F, 600A, 600B, 600C, 600D) of claim 9, further comprising
(i.) detecting at least one of a numeral, an alphabetic character, a fourth symbol, a fifth punctuation mark, in each of the first to eighteenth character strings; and
(ii.) extracting the at least one of the numeral, the alphabetic character, the fourth symbol, the fifth punctuation mark detected in each of the first to eighteenth character strings, to generate the optical prescription value associated with the respective one of the one or more predetermined categories (403, 413, 423, 433, 503, 513, 523, 533, 553, 603, 613, 623, 633), wherein
(a.) the optical prescription value associated with each of the first, second, fourth, and sixth categories has a length less than or equal to 5 characters;
(b.) the optical prescription value associated with the third category has a length less than or equal to 4 characters; and
(c.) the optical prescription value associated with the fifth category has a length less than or equal to 2 characters.

11. The method (700A) of claim 10, wherein determining whether the optical prescription value associated with the respective one of the one or more predetermined categories contains the error, and, if the optical prescription value contains the error, correcting the error within the optical prescription value associated with the respective one of the one or more predetermined categories comprises
(i.) determining if the at least one alphabetic character is present in the optical prescription value associated with the respective one of the one or more predetermined categories (701);
(ii.) if it is determined that the at least one alphabetic character is present in the optical prescription value associated with the respective one of the one or more predetermined categories:
(a.) matching the at least one alphabetic character to one or more predetermined alphabetic character candidates, wherein the one or more predetermined alphabetic character candidates each comprise a corresponding predetermined numeric candidate;
(b.) selecting the corresponding predetermined numeric candidate, by basing the selection on the matching of the at least one alphabetic character to the one or more predetermined alphabetic character candidates; and
(c.) correcting the at least one alphabetic character present in the optical prescription value associated with the respective one of the one or more predetermined categories, to the selected corresponding predetermined numeric candidate (702).

12. The method (700B) of claim 11, further comprising
(i.) determining if the fifth punctuation mark is present in the optical prescription value associated with the respective one of the one or more predetermined categories (711);
(ii.) if it is determined that the fifth punctuation mark is absent in the optical prescription value associated with the respective one of the one or more predetermined categories:
(a.) detecting a last two numerals in the optical prescription value associated with the respective one of the one or more predetermined categories; and
(b.) inserting the fifth punctuation mark prior to the last two numerals of the optical prescription value associated with the respective one of the one or more predetermined categories (712).

13. The method (700C) of claim 12, further comprising
(i.) determining if the last two numerals of the optical prescription value associated with the respective one of the one or more predetermined categories is identical to one or more predetermined numerical strings (721);
(ii.) if it is determined that the last two numerals of the optical prescription value associated with the respective one of the one or more predetermined categories differs to the one or more predetermined numerical strings:
(a.) matching the last two numerals of the optical prescription value associated with the respective one of the one or more predetermined categories to one or more predetermined erroneous numerical strings, wherein the one or more predetermined erroneous numerical strings each comprise a corresponding predetermined numerical string;
(b.) selecting the corresponding predetermined numerical string, by basing the selection on the matching of the last two numerals to the one or more predetermined erroneous numerical strings; and
(c.) correcting the last two numerals in the optical prescription value associated with the respective one of the one or more predetermined categories, to the selected corresponding predetermined numerical string (722);
(iii.) generating the corrected optical prescription value associated with the respective one of the one or more predetermined categories (723).

14. The method (800A) of claim 13, further comprising
(i.) determining if the corrected optical prescription value associated with the respective one of the one or more predetermined categories falls within a reference range of values associated with the respective one of the one or more predetermined categories (801); and
(ii.) emitting a first alert, if it is determined that the corrected optical prescription value falls outside the reference range of values associated with the respective one of the one or more predetermined categories (802);
(iii.) calculating a difference between the corrected optical prescription value associated with the respective one of the one or more predetermined categories with a respective previous optical prescription value associated with the respective one of the one or more predetermined categories (811); and
(iv.) emitting a second alert, if it is determined that the difference is greater than a predefined threshold value associated with the respective one of the one or more predetermined categories (812);
or any combination of the above.

## Patentansprüche

1. Computerimplementiertes Verfahren (100) zum Auslesen einer optischen Verordnung auf einem optischen Verordnungsbild, umfassend
(i.) Detektieren eines die optische Verordnung umfassenden Bereichs auf dem optischen Verordnungsbild (102);
(ii.) Extrahieren der optischen Verordnung und Konvertieren der optischen Verordnung in maschinencodierte optische Verordnungsdaten (104);
(iii.) Klassifizieren eines Teils der optischen Verordnungsdaten in eine oder mehrere vorbestimmte Kategorien, um einen optischen Verordnungswert zu erzeugen, der mit einer jeweiligen der einen oder mehreren vorbestimmten Kategorien assoziiert ist (106); und
(iv.) Bestimmen, ob der mit der jeweiligen der einen oder mehreren vorbestimmten Kategorien assoziierte optische Verordnungswert einen Fehler enthält, der eine falsche Konvertierung eines alphabetischen Zeichens ist, und, falls der optische Verordnungswert den Fehler enthält, Korrigieren des Fehlers innerhalb des optischen Verordnungswerts durch Auswählen eines entsprechenden vorbestimmten Ziffernkandidaten, um einen korrigierten optischen Verordnungswert zu erzeugen, der mit der jeweiligen der einen oder mehreren vorbestimmten Kategorien assoziiert ist (108), wobei
(i.) das Detektieren des die optische Verordnung umfassenden Bereichs auf dem optischen Verordnungsbild (102) Detektieren mindestens eines Schlüsselworts und des mit dem mindestens einen Schlüsselwort assoziierten Teils der optischen Verordnungsdaten umfasst, wobei das Detektieren des mindestens einen Schlüsselworts Detektieren des mindestens einen Schlüsselworts und eines oder mehrerer entsprechender fehlerhafter Schlüsselwörter, die mit einem jeweiligen des mindestens einen Schlüsselworts assoziiert sind, umfasst; und
(ii.) das Extrahieren der optischen Verordnung (102) Extrahieren des mindestens einen Schlüsselworts und des mit dem mindestens einen Schlüsselwort assoziierten Teils der optischen Verordnungsdaten umfasst.

2. Verfahren (100) nach Anspruch 1, wobei die eine oder die mehreren vorbestimmten Kategorien Folgendes umfassen
(i.) eine erste Kategorie, die mit sphärischen Daten einer Person assoziiert ist;
(ii.) eine zweite Kategorie, die mit Zylinderdaten der Person assoziiert ist;
(iii.) eine dritte Kategorie, die mit Zylinderachsendaten der Person assoziiert ist;
(iv.) eine vierte Kategorie, die mit zusätzlichen Linsenstärkedaten der Person assoziiert ist;
(v.) eine fünfte Kategorie, die mit Pupillenabstandsdaten der Person assoziiert ist; und
(vi.) eine sechste Kategorie, die mit axialen Längendaten der Person assoziiert ist.

3. Verfahren (400A) nach Anspruch 2, wobei das Klassifizieren des Teils der optischen Verordnungsdaten in die mit den sphärischen Daten der Person assoziierte erste Kategorie Folgendes umfasst
(i.) Detektieren eines ersten Satzzeichens in den optischen Verordnungsdaten (401); und
(ii.) Extrahieren einer ersten Zeichenfolge, die vor dem ersten Satzzeichen positioniert ist, und einer zweiten Zeichenfolge, die nach dem ersten Satzzeichen positioniert ist, wobei die erste und die zweite Zeichenfolge jeweils eine erste vordefinierte Länge aufweisen (402);
(iii.) Detektieren eines ersten Schlüsselworts aus dem mindestens einen Schlüsselwort, wobei das erste Schlüsselwort mit der ersten Kategorie assoziiert ist (501); und
(iv.) Extrahieren einer neunten Zeichenfolge, die nach dem ersten Schlüsselwort positioniert ist, wobei die neunten Zeichenfolge eine zweite vordefinierte Länge aufweist (502);
(v.) Detektieren eines ersten Symbols und eines ersten Trennzeichens in den optischen Verordnungsdaten (601); und
(vi.) Extrahieren einer dreizehnten Zeichenfolge, die zwischen dem ersten Symbol und dem ersten Trennzeichen positioniert ist (602);
oder eine beliebige Kombination aus dem Obigen, und
wobei die erste vordefinierte Länge 3 Zeichen umfasst und die zweite vordefinierte Länge 7 Zeichen umfasst.

4. Verfahren (400B) nach Anspruch 3, wobei das Klassifizieren des Teils der optischen Verordnungsdaten in die mit den Zylinderdaten der Person assoziierte zweite Kategorie Folgendes umfasst
(i.) Detektieren eines zweiten Satzzeichens in den optischen Verordnungsdaten, wobei das zweite Satzzeichen nach dem ersten Satzzeichen positioniert ist (411); und
(ii.) Extrahieren einer dritten Zeichenfolge, die vor dem zweiten Satzzeichen positioniert ist, und einer vierten Zeichenfolge, die nach dem zweiten Satzzeichen positioniert ist, wobei die dritte und die vierte Zeichenfolge jeweils die erste vordefinierte Länge aufweisen (412);
(iii.) Detektieren einer zehnten Zeichenfolge, die nach dem in die erste Kategorie klassifizierten Teil der optischen Verordnungsdaten positioniert ist (511); und
(iv.) Extrahieren der zehnten Zeichenfolge, wobei die zehnte Zeichenfolge die zweite vordefinierte Länge aufweist (512);
(v.) Detektieren eines zweiten Symbols und eines zweiten Trennzeichens in den optischen Verordnungsdaten, wobei das zweite Symbol und das zweite Trennzeichen nach dem ersten Symbol und dem ersten Trennzeichen positioniert sind (611); und
(vi.) Extrahieren einer vierzehnten Zeichenfolge, die zwischen dem zweiten Symbol und dem zweiten Trennzeichen positioniert ist (612);
oder eine beliebige Kombination aus dem Obigen.

5. Verfahren (400C) nach Anspruch 4, wobei das Klassifizieren des Teils der optischen Verordnung in die mit den zusätzlichen Linsenstärkedaten der Person assoziierte vierte Kategorie Folgendes umfasst
(i.) Detektieren eines dritten Satzzeichens in den optischen Verordnungsdaten, wobei das dritte Satzzeichen nach jedem des ersten und des zweiten Satzzeichens positioniert ist (421); und
(ii.) Extrahieren einer fünften Zeichenfolge, die vor dem dritten Satzzeichen positioniert ist, und einer sechsten Zeichenfolge, die nach dem dritten Satzzeichen positioniert ist, wobei die fünfte und die sechste Zeichenfolge jeweils die erste vordefinierte Länge aufweisen (422);
(iii.) Detektieren eines zweiten Schlüsselworts aus dem mindestens einen Schlüsselwort, wobei das zweite Schlüsselwort mit der vierten Kategorie assoziiert ist (531); und
(iv.) Extrahieren einer elften Zeichenfolge, die nach dem zweiten Schlüsselwort positioniert ist, wobei die elfte Zeichenfolge die zweite vordefinierte Länge aufweist (532);
oder eine beliebige Kombination aus dem Obigen.

6. Verfahren (400D) nach Anspruch 5, wobei das Klassifizieren des Teils der optischen Verordnungsdaten in die mit den axialen Längendaten der Person assoziierte sechste Kategorie Folgendes umfasst
(i.) Detektieren eines vierten Satzzeichens in den optischen Verordnungsdaten, wobei das vierte Satzzeichen nach jedem des ersten, des zweiten und des dritten Satzzeichens positioniert ist (431); und
(ii.) Extrahieren einer siebten Zeichenfolge, die vor dem vierten Satzzeichen positioniert ist, und einer achten Zeichenfolge, die nach dem vierten Satzzeichen positioniert ist, wobei die siebte und die achte Zeichenfolge jeweils 2 Zeichen umfassen (432);
(iii.) Detektieren eines dritten Schlüsselworts aus dem mindestens einen Schlüsselwort, wobei das dritte Schlüsselwort mit der sechsten Kategorie assoziiert ist (551);
(iv.) Extrahieren einer zwölften Zeichenfolge, die nach dem dritten Schlüsselwort positioniert ist, wobei die zwölfte Zeichenfolge die zweite vordefinierte Länge aufweist (552); und ferner umfassend
(v.) Bestimmen, ob eine Einheit, die mit der sechsten Kategorie assoziiert ist, in der zwölften Zeichenfolge vorhanden ist;
(vi.) falls bestimmt wird, dass die mit der sechsten Kategorie assoziierte Einheit in der zwölften Zeichenfolge vorhanden ist: Extrahieren einer zwanzigsten Zeichenfolge, die vor der mit der sechsten Kategorie assoziierten Einheit positioniert ist, um den mit der sechsten Kategorie assoziierten optischen Verordnungswert zu erzeugen (554);
oder eine beliebige Kombination aus dem Obigen.

7. Verfahren (600C) nach einem der Ansprüche 4 bis 6, wobei das Klassifizieren des Teils der optischen Verordnungsdaten in die mit den Zylinderachsendaten der Person assoziierte dritte Kategorie Folgendes umfasst
(i.) Detektieren eines dritten Trennzeichens in den optischen Verordnungsdaten, wobei das dritte Trennzeichen nach jedem des ersten und des zweiten Trennzeichens positioniert ist (621); und
(ii.) Extrahieren einer fünfzehnten Zeichenfolge, die nach dem dritten Trennzeichen positioniert ist (622);
(iii.) Detektieren einer siebzehnten Zeichenfolge, die nach dem in die zweite Kategorie klassifizierten Teil der optischen Verordnungsdaten positioniert ist (521); und
(iv.) Extrahieren der siebzehnten Zeichenfolge, wobei die siebzehnte Zeichenfolge 6 Zeichen umfasst (522);
oder eine beliebige Kombination aus dem Obigen.

8. Verfahren (600D) nach Anspruch 7, wobei das Klassifizieren des Teils der optischen Verordnung in die mit den zusätzlichen Linsenstärkedaten der Person assoziierte vierte Kategorie Folgendes umfasst
(i.) Detektieren eines dritten Symbols und eines vierten Trennzeichens in den optischen Verordnungsdaten, wobei das dritte Symbol und das vierte Trennzeichen nach jedem des ersten und des zweiten Symbols und jedem des ersten bis dritten Trennzeichens positioniert sind (631);
(ii.) Extrahieren einer sechzehnten Zeichenfolge, die zwischen dem dritten Symbol und dem vierten Trennzeichen positioniert ist (632).

9. Verfahren (500E) nach einem der Ansprüche 2 bis 8, wobei das Klassifizieren des Teils der optischen Verordnung in die mit den Pupillenabstandsdaten der Person assoziierte fünfte Kategorie Folgendes umfasst
(i.) Detektieren eines vierten Schlüsselworts aus dem mindestens einen Schlüsselwort, wobei das vierte Schlüsselwort mit der fünften Kategorie assoziiert ist (541);
(ii.) Extrahieren einer achtzehnten Zeichenfolge, die nach dem vierten Schlüsselwort positioniert ist, wobei die achtzehnte Zeichenfolge 5 Zeichen umfasst (542);
(iii.) Bestimmen, ob eine Einheit, die mit der fünften Kategorie assoziiert ist, in der achtzehnten Zeichenfolge vorhanden ist;
(iv.) falls bestimmt wird, dass die mit der fünften Kategorie assoziierte Einheit in der achtzehnten Zeichenfolge vorhanden ist:
Extrahieren einer neunzehnten Zeichenfolge, die vor der mit der fünften Kategorie assoziierten Einheit positioniert ist, um den mit der fünften Kategorie assoziierten optischen Verordnungswert zu erzeugen (543).

10. Verfahren (400A, 400B, 400C, 400D, 500A, 500B, 500C, 500D, 500F, 600A, 600B, 600C, 600D) nach Anspruch 9, ferner umfassend
(i.) Detektieren mindestens eines einer Ziffer, eines alphabetischen Zeichens, eines vierten Symbols, eines fünften Satzzeichens in jeder der ersten bis achtzehnten Zeichenfolge; und
(ii.) Extrahieren des mindestens einen der Ziffer, des alphabetischen Zeichens, des vierten Symbols, des fünften Satzzeichens, die/das in jeder der ersten bis achtzehnten Zeichenfolge detektiert wird, um den mit der jeweiligen der einen oder mehreren vorbestimmten Kategorien assoziierten optischen Verordnungswert zu erzeugen (403, 413, 423, 433, 503, 513, 523, 533, 553, 603, 613, 623, 633), wobei
(a.) der mit jeder der ersten, der zweiten, der vierten und der sechsten Kategorie assoziierte optische Verordnungswert eine Länge von weniger als oder gleich 5 Zeichen aufweist;
(b.) der mit der dritten Kategorie assoziierte optische Verordnungswert eine Länge von weniger als oder gleich 4 Zeichen aufweist; und
(c.) der mit der fünften Kategorie assoziierte optische Verordnungswert eine Länge von weniger als oder gleich 2 Zeichen aufweist.

11. Verfahren (700A) nach Anspruch 10, wobei das Bestimmen, ob der mit der jeweiligen der einen oder mehreren vorbestimmten Kategorien assoziierte optische Verordnungswert den Fehler enthält, und, falls der optische Verordnungswert den Fehler enthält, das Korrigieren des Fehlers innerhalb des mit der jeweiligen der einen oder mehreren vorbestimmten Kategorien assoziierten optischen Verordnungswerts Folgendes umfasst
(i.) Bestimmen, ob das mindestens eine alphabetische Zeichen in dem mit der jeweiligen der einen oder mehreren vorbestimmten Kategorien assoziierten optischen Verordnungswert vorhanden ist (701);
(ii.) falls bestimmt wird, dass das mindestens eine alphabetische Zeichen in dem mit der jeweiligen der einen oder mehreren vorbestimmten Kategorien assoziierten optischen Verordnungswert vorhanden ist:
(a.) Abgleichen des mindestens einen alphabetischen Zeichens mit einem oder mehreren vorbestimmten alphabetischen Zeichenkandidaten, wobei der eine oder die mehreren vorbestimmten alphabetischen Zeichenkandidaten jeweils einen entsprechenden vorbestimmten Ziffernkandidaten umfassen;
(b.) Auswählen des entsprechenden vorbestimmten Ziffernkandidaten durch Basieren der Auswahl auf das Abgleichen des mindestens einen alphabetischen Zeichens mit dem einen oder den mehreren vorbestimmten alphabetischen Zeichenkandidaten; und
(c.) Korrigieren des mindestens einen alphabetischen Zeichens, das in dem mit der jeweiligen der einen oder mehreren vorbestimmten Kategorien assoziierten optischen Verordnungswert vorhanden ist, auf den ausgewählten entsprechenden vorbestimmten Ziffernkandidaten (702).

12. Verfahren (700B) nach Anspruch 11, ferner umfassend
(i.) Bestimmen, ob das fünfte Satzzeichen in dem mit der jeweiligen der einen oder mehreren vorbestimmten Kategorien assoziierten optischen Verordnungswert vorhanden ist (711);
(ii.) falls bestimmt wird, dass das fünfte Satzzeichen in dem mit der jeweiligen der einen oder mehreren vorbestimmten Kategorien assoziierten optischen Verordnungswert nicht vorhanden ist:
(a.) Detektieren der letzten zwei Ziffern in dem mit der jeweiligen der einen oder mehreren vorbestimmten Kategorien assoziierten optischen Verordnungswert; und
(b.) Einfügen des fünften Satzzeichens vor den letzten zwei Ziffern des mit der jeweiligen der einen oder mehreren vorbestimmten Kategorien assoziierten optischen Verordnungswerts (712).

13. Verfahren (700C) nach Anspruch 12, ferner umfassend
(i.) Bestimmen, ob die letzten zwei Ziffern des mit der jeweiligen der einen oder mehreren vorbestimmten Kategorien assoziierten optischen Verordnungswerts mit einer oder mehreren vorbestimmten Ziffernfolgen identisch sind (721);
(ii.) falls bestimmt wird, dass sich die letzten zwei Ziffern des mit der jeweiligen der einen oder mehreren vorbestimmten Kategorien assoziierten optischen Verordnungswerts von der einen oder den mehreren vorbestimmten Ziffernfolgen unterscheiden:
(a.) Abgleichen der letzten zwei Ziffern des mit der jeweiligen der einen oder mehreren vorbestimmten Kategorien assoziierten optischen Verordnungswerts mit einer oder mehreren vorbestimmten fehlerhaften Ziffernfolgen, wobei die eine oder die mehreren vorbestimmten fehlerhaften Ziffernfolgen jeweils eine entsprechende vorbestimmte Ziffernfolge umfassen;
(b.) Auswählen der entsprechenden vorbestimmten Ziffernfolge durch Basieren der Auswahl auf das Abgleichen der letzten zwei Ziffern mit der einen oder den mehreren vorbestimmten fehlerhaften Ziffernfolgen; und
(c.) Korrigieren der letzten zwei Ziffern in dem mit der jeweiligen der einen oder mehreren vorbestimmten Kategorien assoziierten optischen Verordnungswert auf die ausgewählte entsprechende vorbestimmte Ziffernfolge (722);
(iii.) Erzeugen des korrigierten optischen Verordnungswerts, der mit der jeweiligen der einen oder mehreren vorbestimmten Kategorien assoziiert ist (723).

14. Verfahren (800A) nach Anspruch 13, ferner umfassend (i.) Bestimmen, ob der mit der jeweiligen der einen oder mehreren vorbestimmten Kategorien assoziierte korrigierte optische Verordnungswert innerhalb eines Referenzbereichs von Werten liegt, der mit der jeweiligen der einen oder mehreren vorbestimmten Kategorien assoziiert ist (801); und
(ii.) Ausgeben einer ersten Warnung, falls bestimmt wird, dass der korrigierte optische Verordnungswert außerhalb des mit der jeweiligen der einen oder mehreren vorbestimmten Kategorien assoziierten Referenzbereichs von Werten liegt (802);
(iii.) Berechnen einer Differenz zwischen dem mit der jeweiligen der einen oder mehreren vorbestimmten Kategorien assoziierten korrigierten optischen Verordnungswert und einem jeweiligen vorherigen optischen Verordnungswert, der mit der jeweiligen der einen oder mehreren vorbestimmten Kategorien assoziiert ist (811); und
(iv.) Ausgeben einer zweiten Warnung, falls bestimmt wird, dass die Differenz größer als ein vordefinierter Schwellenwert ist, der mit der jeweiligen der einen oder mehreren vorbestimmten Kategorien assoziiert ist (812); oder eine beliebige Kombination aus dem Obigen.

## Revendications

1. Procédé mis en œuvre par ordinateur (100) pour lire une prescription optique sur une image de prescription optique comprenant
(i.) la détection d'une région comprenant la prescription optique sur l'image de prescription optique (102) ;
(ii.) l'extraction de la prescription optique et la conversion de la prescription optique en données de prescription optique codées par machine (104) ;
(iii.) la classification d'une partie des données de prescription optique dans une ou plusieurs catégories prédéterminées, pour générer une valeur de prescription optique associée à une catégorie respective parmi la ou les catégories prédéterminées (106) ; et
(iv.) la détermination pour savoir si la valeur de prescription optique associée à la catégorie respective parmi la ou les catégories prédéterminées contient une erreur qui est une conversion incorrecte d'un caractère alphabétique, et, si la valeur de prescription optique contient l'erreur, la correction de l'erreur à l'intérieur de la valeur de prescription optique en sélectionnant un candidat numérique prédéterminé correspondant, pour générer une valeur de prescription optique corrigée associée à la catégorie respective parmi la ou les catégories prédéterminées (108), dans lequel
(i.) la détection de la région comprenant la prescription optique sur l'image de prescription optique (102) comprend la détection d'au moins un mot-clé et de la partie des données de prescription optique associées au ou aux mots-clés, la détection du ou des mots-clés comprenant la détection du ou des mots-clés et d'un ou plusieurs mots-clés erronés correspondants associés à un mot-clé respectif parmi le ou les mots-clés ; et
(ii.) l'extraction de la prescription optique (102) consiste à extraire le ou les mots-clés et la partie des données de prescription optique associées au ou aux mots-clés.

2. Procédé (100) selon la revendication 1, dans lequel la ou les catégories prédéterminées comprennent
(i.) une première catégorie associée aux données sphériques d'un sujet ;
(ii.) une deuxième catégorie associée aux données cylindriques d'un sujet ;
(iii.) une troisième catégorie associée aux données d'un axe cylindrique d'un sujet ;
(iv.) une quatrième catégorie associée aux données de puissance d'objectif supplémentaires d'un sujet ;
(v.) une cinquième catégorie associée aux données de distance pupillaire d'un sujet ; et
(vi.) une sixième catégorie associée aux données de longueur axiale d'un sujet.

3. Procédé (400A) selon la revendication 2, dans lequel la classification de la partie des données de prescription optique dans la première catégorie associée aux données sphériques d'un sujet comprend
(i.) la détection d'un premier signe de ponctuation dans les données de prescription optique (401) ; et
(ii.) l'extraction d'une première chaîne de caractères positionnée avant le premier signe de ponctuation et d'une deuxième chaîne de caractères positionnée après le premier signe de ponctuation, les première et deuxième chaînes de caractères ayant chacune une première longueur prédéfinie (402) ;
(iii.) la détection d'un premier mot-clé parmi le ou les mots-clés, le premier mot-clé étant associé à la première catégorie (501) ; et
(iv.) l'extraction d'une neuvième chaîne de caractères positionnée après le premier mot-clé, la neuvième chaîne de caractères ayant une seconde longueur prédéfinie (502) ;
(v.) la détection d'un premier symbole et d'un premier séparateur dans les données de prescription optique (601) ; et
(vi.) l'extraction d'une treizième chaîne de caractères positionnée entre le premier symbole et le premier séparateur (602) ;
ou toute combinaison des éléments ci-dessus, et
dans lequel la première longueur prédéfinie comprend 3 caractères et la seconde longueur prédéfinie comprend 7 caractères.

4. Procédé (400B) selon la revendication 3, dans lequel la classification de la partie des données de prescription optique dans la deuxième catégorie associée aux données cylindriques d'un sujet comprend
(i.) la détection d'un deuxième signe de ponctuation dans les données de prescription optique, le deuxième signe de ponctuation étant positionné après le premier signe de ponctuation (411) ; et
(ii.) l'extraction d'une troisième chaîne de caractères positionnée avant le deuxième signe de ponctuation, et d'une quatrième chaîne de caractères positionnée après le deuxième signe de ponctuation, les troisième et quatrième chaînes de caractères ayant chacune la première longueur prédéfinie (412) ;
(iii.) la détection d'une dixième chaîne de caractères positionnée après la partie des données de prescription optique classée dans la première catégorie (511) ; et (iv.) l'extraction de la dixième chaîne de caractères, la dixième chaîne de caractères ayant la seconde longueur prédéfinie (512) ;
(v) la détection d'un deuxième symbole et d'un deuxième séparateur dans les données de prescription optique, le deuxième symbole et le deuxième séparateur étant positionnés après le premier symbole et le premier séparateur (611) ; et
(vi.) l'extraction d'une quatorzième chaîne de caractères positionnée entre le deuxième symbole et le deuxième séparateur (612) ;
ou toute combinaison des éléments ci-dessus.

5. Procédé (400C) selon la revendication 4, dans lequel la classification de la partie de la prescription optique dans la quatrième catégorie associée aux données de puissance de puissance d'objectif supplémentaires d'un sujet comprend
(i.) la détection d'un troisième signe de ponctuation dans les données de prescription optique, le troisième signe de ponctuation étant positionné après chacun des premier et deuxième signes de ponctuation (421) ; et
(ii.) l'extraction d'une cinquième chaîne de caractères positionnée avant le troisième signe de ponctuation, et d'une sixième chaîne de caractères positionnée après le troisième signe de ponctuation, les cinquième et sixième chaînes de caractères ayant chacune la première longueur prédéfinie (422) ;
(iii.) la détection d'un deuxième mot-clé parmi le ou les mots-clés, le deuxième mot-clé étant associé à la quatrième catégorie (531) ; et
(iv.) l'extraction d'une onzième chaîne de caractères positionnée après le deuxième mot-clé, la onzième chaîne de caractères ayant la seconde longueur prédéfinie (532) ;
ou toute combinaison des éléments ci-dessus.

6. Procédé (400D) selon la revendication 5, dans lequel la classification de la partie des données de prescription optique dans la sixième catégorie associée aux données de longueur axiale d'un sujet comprend
(i.) la détection d'un quatrième signe de ponctuation dans les données de prescription optique, le quatrième signe de ponctuation étant positionné après chacun des premier, deuxième et troisième signes de ponctuation (431) ; et
(ii.) l'extraction d'une septième chaîne de caractères positionnée avant le quatrième signe de ponctuation et d'une huitième chaîne de caractères positionnée après le quatrième signe de ponctuation, les septième et huitième chaînes de caractères comprenant chacune 2 caractères (432) ;
(iii.) la détection d'un troisième mot-clé parmi le ou les mots-clés, le troisième mot-clé étant associé à la sixième catégorie (551) ;
(iv.) l'extraction d'une douzième chaîne de caractères positionnée après le troisième mot-clé, la douzième chaîne de caractères ayant la seconde longueur prédéfinie (552) ; et comprenant en outre
v.) la détermination pour savoir si une unité associée à la sixième catégorie est présente dans la douzième chaîne de caractères ;
vi.) si l'on détermine que l'unité associée à la sixième catégorie est présente dans la douzième chaîne de caractères : extraire une vingtième chaîne de caractères positionnée avant l'unité associée à la sixième catégorie, pour générer la valeur de prescription optique associée à la sixième catégorie (554) ;
ou toute combinaison des éléments ci-dessus.

7. Procédé (600C) selon l'une quelconque des revendications 4 à 6, dans lequel la classification de la partie des données de prescription optique dans la troisième catégorie associée aux données d'axe de cylindre d'un sujet comprend
(i.) la détection d'un troisième séparateur dans les données de prescription optique, le troisième séparateur étant positionné après chacun des premier et deuxième séparateurs (621) ; et
(ii.) l'extraction d'une quinzième chaîne de caractères positionnée après le troisième séparateur (622) ;
(iii.) la détection d'une dix-septième chaîne de caractères positionnée après la partie des données de prescription optique classée dans la deuxième catégorie (521) ; et
(iv.) l'extraction de la dix-septième chaîne de caractères, la dix-septième chaîne de caractères comprenant 6 caractères (522) ;
ou toute combinaison des éléments ci-dessus.

8. Procédé (600D) selon la revendication 7, dans lequel la classification de la partie de la prescription optique dans la quatrième catégorie associée aux données de puissance d'objectif supplémentaires d'un sujet comprend (i.) la détection d'un troisième symbole et d'un quatrième séparateur dans les données de prescription optique, le troisième symbole et le quatrième séparateur étant positionnés après chacun des premier et deuxième symboles et chacun des premier à troisième séparateurs (631) ;
(ii.) l'extraction d'une seizième chaîne de caractères positionnée entre le troisième symbole et le quatrième séparateur (632).

9. Procédé (500E) selon l'une quelconque des revendications 2 à 8, dans lequel la classification de la partie de la prescription optique dans la cinquième catégorie associée aux données de distance pupillaire d'un sujet comprend
(i.) la détection d'un quatrième mot-clé parmi le ou les mots-clés, le quatrième mot-clé étant associé à la cinquième catégorie (541) ;
(ii.) l'extraction d'une dix-huitième chaîne de caractères positionnée après le quatrième mot-clé, la dix-huitième chaîne de caractères comprenant 5 caractères (542) ;
iii.) la détermination pour savoir si une unité associée à la cinquième catégorie est présente dans la dix-huitième chaîne de caractères ;
(iv.) s'il est déterminé que l'unité associée à la cinquième catégorie est présente dans la dix-huitième chaîne de caractères :
l'extraction d'une dix-neuvième chaîne de caractères positionnée avant l'unité associée à la cinquième catégorie, pour générer la valeur de prescription optique associée à la cinquième catégorie (543).

10. Procédé (400A, 400B, 400C, 400D, 500A, 500B, 500C, 500D, 500F, 600A, 600B, 600C, 600D) selon la revendication 9, comprenant en outre
(i.) la détection d'au moins l'un parmi un chiffre, un caractère alphabétique, un quatrième symbole, un cinquième signe de ponctuation, dans chacune des première à dix-huitième chaînes de caractères ; et
(ii.) l'extraction de l'au moins un parmi le chiffre, le caractère alphabétique, le quatrième symbole, le cinquième signe de ponctuation détecté dans chacune des première à dix-huitième chaînes de caractères pour générer la valeur de prescription optique associée à la catégorie respective parmi la ou les catégories prédéterminées (403, 413, 423, 433, 503, 513, 523, 533, 553, 603, 613, 623, 633), dans lequel
(a.) la valeur de prescription optique associée à chacune des première, deuxième, quatrième et sixième catégories a une longueur inférieure ou égale à 5 caractères ;
(b.) la valeur de prescription optique associée à la troisième catégorie a une longueur inférieure ou égale à 4 caractères ; et
(c.) la valeur de prescription optique associée à la cinquième catégorie a une longueur inférieure ou égale à 2 caractères.

11. Procédé (700A) selon la revendication 10, dans lequel la détermination pour savoir si la valeur de prescription optique associée à la catégorie respective parmi la ou les catégories prédéterminées contient l'erreur, et, si la valeur de prescription optique contient l'erreur, la correction de l'erreur à l'intérieur de la valeur de prescription optique associée à la catégorie respective parmi la ou les catégories prédéterminées comprend
(i.) la détermination pour savoir si la ou les caractères alphabétiques sont présents dans la valeur de prescription optique associée à la catégorie respective parmi la ou les catégories prédéterminées (701) ;
(ii.) s'il est déterminé que le ou les caractères alphabétiques sont présents dans la valeur de prescription optique associée à la catégorie respective parmi la ou des catégories prédéterminées :
(a.) la mise en correspondance du ou des caractères alphabétiques avec un ou plusieurs candidats à caractère alphabétique prédéterminés, le ou les candidats à caractère alphabétique prédéterminés comprenant chacun un candidat numérique prédéterminé correspondant ;
(b.) la sélection du candidat numérique prédéterminé correspondant, en basant la sélection sur la mise en correspondance entre le ou les caractères alphabétiques et le ou les candidats à caractère alphabétique prédéterminés ; et
(c.) la correction du ou des caractères alphabétiques présents dans la valeur de prescription optique associée à la catégorie respective parmi la ou les catégories prédéterminées, au candidat numérique prédéterminé correspondant sélectionné (702).

12. Procédé (700B) selon la revendication 11, comprenant en outre
(i.) la détermination pour savoir si la cinquième marque de ponctuation est présente dans la valeur de prescription optique associée à la catégorie respective parmi la ou des catégories prédéterminées (711) ;
(ii.) s'il est déterminé que le cinquième signe de ponctuation est absent dans la valeur de prescription optique associée à la catégorie respective parmi la ou les catégories prédéterminées :
(a.) la détection des deux derniers chiffres dans la valeur de prescription optique associée à la catégorie respective parmi la ou les catégories prédéterminées ; et
(b.) l'insertion du cinquième signe de ponctuation antérieur aux deux derniers chiffres de la valeur de prescription optique associée à la catégorie respective parmi la ou les catégories prédéterminées (712).

13. Procédé (700C) selon la revendication 12, comprenant en outre
(i.) la détermination pour savoir si les deux derniers chiffres de la valeur de prescription optique associée à la catégorie respective parmi la ou les catégories prédéterminées sont identiques à une ou de plusieurs chaînes numériques prédéterminées (721) ;
(ii.) s'il est déterminé que les deux derniers chiffres de la valeur de prescription optique associée à la catégorie respective parmi la ou les catégories prédéterminées diffèrent de la ou des chaînes numériques prédéterminées :
(a.) la mise en correspondance des deux derniers chiffres de la valeur de prescription optique associée à la catégorie respective parmi la ou les catégories prédéterminées avec une ou de plusieurs chaînes numériques erronées prédéterminées, la ou les chaînes numériques erronées prédéterminées comprenant chacune une chaîne numérique prédéterminée correspondante ;
(b.) la sélection de la chaîne numérique prédéterminée correspondante, en basant la sélection sur la mise en correspondance des deux derniers chiffres avec la ou les chaînes numériques erronées prédéterminées ; et
(c.) la correction des deux derniers chiffres de la valeur de prescription optique associée à la catégorie respective parmi la ou les catégories prédéterminées, à la chaîne numérique prédéterminée correspondante sélectionnée (722) ;
(iii.) la génération de la valeur de prescription optique corrigée associée à la catégorie respective parmi la ou les catégories prédéterminées (723).

14. Procédé (800A) selon la revendication 13, comprenant en outre
(i.) la détermination pour savoir si la valeur de prescription optique corrigée associée à la catégorie respective parmi la ou les catégories prédéterminées entre dans une plage de référence de valeurs associées à la catégorie respective parmi la ou les catégories prédéterminées (801) ; et
(ii.) l'émission d'une première alerte, s'il est déterminé que la valeur de prescription optique corrigée sort de la plage de référence de valeurs associée à la catégorie respective parmi la ou les catégories prédéterminées (802) ;
(iii.) le calcul d'une différence entre la valeur de prescription optique corrigée associée à la catégorie respective parmi la ou les catégories prédéterminées avec une valeur de prescription optique précédente respective associée à la catégorie respective parmi la ou les catégories prédéterminées (811) ; et
(iv.) l'émission d'une seconde alerte, s'il est déterminé que la différence est supérieure à une valeur seuil prédéfinie associée à la catégorie respective parmi la ou les catégories prédéterminées (812) ;
ou toute combinaison des éléments ci-dessus.
